(19) Europäisches Patentamt
European Patent Office
Office européen des brevets

(11) **EP 3 090 052 B1**

(12) **EUROPEAN PATENT SPECIFICATION**

(45) Date of publication and mention of the grant of the patent:
**13.06.2018 Bulletin 2018/24**

(21) Application number: **14830949.5**

(22) Date of filing: **30.12.2014**

(51) Int Cl.:
***C12N 15/67*** (2006.01) ***C12N 15/85*** (2006.01)

(86) International application number:
**PCT/EP2014/003481**

(87) International publication number:
**WO 2015/101415 (09.07.2015 Gazette 2015/27)**

(54) **ARTIFICIAL NUCLEIC ACID MOLECULES**

KÜNSTLICHE NUKLEINSÄUREMOLEKÜLE

MOLÉCULES D'ACIDES NUCLÉIQUES ARTIFICIELLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2013 PCT/EP2013/003948**

(43) Date of publication of application:
**09.11.2016 Bulletin 2016/45**

(73) Proprietor: **CureVac AG**
**72076 Tübingen (DE)**

(72) Inventors:
- **GRUND, Stefanie**
  **72076 Tübingen (DE)**
- **SCHLAKE, Thomas**
  **79194 Gundelfingen (DE)**

(74) Representative: **Graf von Stosch, Andreas et al**
**Graf von Stosch Patentanwaltsgesellschaft mbH**
**Prinzregentenstraße 22**
**80538 München (DE)**

(56) References cited:
**WO-A1-2008/134539 WO-A1-2013/120629**
**WO-A1-2013/143698 WO-A2-2008/112127**
**WO-A2-2013/143700**

- **DATABASE GenBank [Online] NCBI; 6 August 2014 (2014-08-06), "Homosapiens FIG4 phosphoinositide 5-phosphatase", XP002729573, Database accession no. NM_014845.5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 090 052 B1

## Description

The invention relates to a pharmaceutical composition comprising artificial nucleic acid molecules comprising an open reading frame, a 3'-untranslated region element (3'-UTR element) as defined in the claims and optionally a poly(A) sequence and/or a polyadenylation-signal. The invention relates further to a kit comprising the pharmaceutical composition. Furthermore, the invention provides the pharmaceutical composition and the kit for use in the field of gene therapy and/or genetic vaccination. In addition, an in vitro method for stabilizing and/or prolonging protein production from an artificial nucleic acid molecule as defined in the claims is provided. The invention further concerns the use of an 3'-UTR element for stabilizing and/or prolonging protein production from a nucleic acid as defined in the claims.

Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent early onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to misregulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows evoking a desired immune response to selected antigens, such as characteristic components of bacterial surfaces, viral particles, tumour antigens or the like. Generally, vaccination is one of the pivotal achievements of modern medicine. However, effective vaccines are currently available only for a limited number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein antigen fragments characteristic for a pathogen or a tumor antigen *in vivo.* Genetic vaccines are expressed upon administration to a patient after uptake by target cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *ex vivo* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting in mutagenic events such as loss of function of the impaired genes. As a further risk, the undesired generation of anti-DNA antibodies has emerged. Another drawback is the limited expression level of the encoded peptide or protein that is achievable upon DNA administration because the DNA must enter the nucleus in order to be transcribed before the resulting mRNA can be translated. Among other reasons, the expression level of the administered DNA will be dependent on the presence of specific transcription factors which regulate DNA transcription. In the absence of such factors, DNA transcription will

not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

*In vivo,* RNA degradation contributes to the regulation of the RNA half-life time. That effect was considered and proven to fine tune the regulation of eukaryotic gene expression (Friedel et al., 2009. Conserved principles of mammalian transcriptional regulation revealed by RNA half-life, Nucleic Acid Research 37(17): 1-12). Accordingly, each naturally occurring mRNA has its individual half-life depending on the gene from which the mRNA is derived and in which cell type it is expressed. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half-life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that the product encoded by the RNA sequence shall accumulate *in vivo.* On the other hand, the RNA has to maintain its structural and functional integrity when prepared for a suitable dosage form, in the course of its storage, and when administered. Thus, considerable attention was dedicated to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the coding region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'-UTR) and/or at their 3'-end (3'-UTR) as well as other structural features, such as a 5'-cap structure or a 3'-poly(A) tail. Both, 5'-UTR and 3'-UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenosine nucleotides added to the 3'-end of the transcribed mRNA. It may comprise up to about 400 adenosine nucleotides. It was found that the length of such a 3'-poly(A) tail is a potentially critical element for the stability of the individual mRNA.

International patent application WO2008/112127 describes regulatory sequences operably linked with a reporter gene and a 5'-UTR, a 3'-UTR or both, wherein the translation of the reporter gene is under regulatory control of control regions in the UTR sequence. In particular, libraries of expression constructs are described therein, which have different UTR sequences derived from a genome.

Nucleic acid constructs for increasing the expression of an encoded protein are also described in International Patent application WO2013/120629. The nucleic acids described therein comprise or code for a histone stem-loop and a poly(A) sequence or a polyadenylation signal.

International patent application WO2008/134539 describes the FIG4 gene, mutations thereof as well as assays for the detection of variant FIG4 alleles, polymorphisms and mutations.

Also, it was shown that the 3'-UTR of α-globin mRNA may be an important factor for the well-known stability of α-globin mRNA (Rodgers et a/., Regulated α-globin mRNA decay is a cytoplasmic event proceeding through 3'-to-5' exosome-dependent decapping, RNA, 8, pp. 1526-1537, 2002). The 3'-UTR of α-globin mRNA is obviously involved in the formation of a specific ribonucleoprotein-complex, the α-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

Irrespective of factors influencing mRNA stability, effective translation of the administered nucleic acid molecules by the target cells or tissue is crucial for any approach using nucleic acid molecules for gene therapy or genetic vaccination. Along with the regulation of stability, also translation of the majority of mRNAs is regulated by structural features like UTRs, 5'-cap and 3'-poly(A) tail. In this context, it has been reported that the length of the poly(A) tail may play an

important role for translational efficiency as well. Stabilizing 3'-elements, however, may also have an attenuating effect on translation.

It is the object of the invention to provide nucleic acid molecules which may be suitable for application in gene therapy and/or genetic vaccination. Particularly, it is the object of the invention to provide an mRNA species which is stabilized against preterm degradation or decay without exhibiting significant functional loss in translational efficiency. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject matter.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigens. In the context of the present invention, tumour antigens

and pathogenic antigens as defined herein are particularly preferred.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Bicistronic RNA multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions; particularly under physiological conditions *in vivo.* A "cationic peptide or protein" may contain at least one positively charged amino acid, or more than one positively charged amino acid, e.g. selected from Arg, His, Lys or Orn. Accordingly, "polycationic" components are also within the scope exhibiting more than one positive charge under the conditions given.

5'-cap: A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic-acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first strand typically hybridize with the nucleotides of the second strand, e.g. by A/T-base-pairing and G/C-base-pairing.

Epitope: (also called "antigen determinant") can be distinguished in T cell epitopes and B cell epitopes. T cell

epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivolex vivo or in vitro;* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro;* b) transcription and/or translation of the introduced nucleic acid molecule; and optionally c) re-administration of isolated, transfected cells to the subject, preferably the patient, if the nucleic acid has not been administered directly to the patient. Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention an immunogen may be typically understood to be a compound that is able to stimulate an immune response. Preferably, an immunogen is a peptide, polypeptide, or protein. In a

particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG), at its 5'- end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein: A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenosine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA. mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites. The 5'-UTR may be post-transcriptionally modified, for example by addition of a 5'-CAP. In the context of the present invention, a 5'-UTR corresponds to the sequence of a mature mRNA which is located between the 5'-CAP and the start codon. Preferably, the 5'-UTR corresponds to the sequence which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such

as in the mRNA sequence used for defining the 5'-UTR sequence, or a DNA sequence which corresponds to such RNA sequence. In the context of the present invention, the term "a 5'-UTR of a gene" is the sequence which corresponds to the 5'-UTR of the mature mRNA derived from this gene, i.e. the mRNA obtained by transcription of the gene and maturation of the pre-mature mRNA.

The term "5'-UTR of a gene" encompasses the DNA sequence and the RNA sequence of the 5'-UTR. Such a 5'-UTR may be provided 5'-terminal to the ORF. Its length is typically less than 500, 400, 300, 250 or less than 200 nucleotides. In other embodiments its length may be in the range of at least 10, 20, 30 or 40, preferably up to 100 or 150, nucleotides.

5'Terminal Oligopyrimidine Tract (TOP): The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purine residue in said gene or mRNA. A TOP motif in the sense of the present invention is preferably located at the 5'end of a sequence which represents a 5'UTR or at the 5'end of a sequence which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule, the 5'UTR element of the artificial nucleic acid molecule, or the nucleic acid sequence which is derived from the 5'UTR of a TOP gene as described herein. In other words, a stretch of 3 or more pyrimidine nucleotides, which is not located at the 5'-end of a 5'UTR or a 5'UTR element but anywhere within a 5'UTR or a 5'UTR element, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'-CAP to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID Nos. 1-1363 of the patent application WO2013/143700. In this context a particularly preferred fragment of a 5'UTR of a TOP gene is a 5'UTR of a TOP gene lacking the 5'TOP motif. The terms "5'UTR of a TOP gene" or "5'-TOP UTR" preferably refer to the 5'UTR of a naturally occurring TOP gene.

3'-untranslated region (3'-UTR): A 3'-UTR is typically the part of an mRNA which is located between the protein coding region (open reading frame (ORF) or coding sequence (CDS)) and the poly(A) sequence of the mRNA. A 3'-UTR of the mRNA is not translated into an amino acid sequence. The 3'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'- end of the pre-mature mRNA and optional endo- or exonuclease cleavages etc. In the context of the present invention, a 3'-UTR corresponds to the sequence of a mature mRNA which is located 3' to the stop codon of the protein coding region (open reading frame), preferably immediately 3' to the stop codon of the protein coding region, and which extends to the 5'-side of the poly(A) sequence, preferably to the nucleotide immediately 5' to the poly(A) sequence. The term "corresponds

to" means that the 3'-UTR sequence may be an RNA sequence, such as in the mRNA sequence ("sense mRNA) used for defining the 3'-UTR sequence, a DNA sequence which corresponds to such RNA sequence or a RNA or DNA sequence which is complementary to such an RNA sequence ("antisense RNA"). In the context of the present invention, the term "a 3'-UTR of a gene", such as "a 3'-UTR of a FIG4 gene", is the sequence which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence of the 3'-UTR.

The pharmaceutical composition according to the invention comprises an artificial nucleic acid molecule comprising

a. at least one open reading frame (ORF); and
b. at least one 3'-untranslated region element (3'-UTR element) comprising or consisting of a nucleic acid sequence which is derived from the 3'-UTR of a FIG4 gene or from a variant of the 3'-UTR of a FIG4 gene,

wherein the open reading frame (ORF) and the 3'-UTR element are heterologous to each other. In the context of the present invention, the "nucleic acid sequence which is derived from the 3'UTR of a FIG4 gene (or from a variant of the 3'-UTR of a FIG4 gene)" is a nucleic acid sequence as defined in the claims, i.e. a nucleic acid sequence according to SEQ ID NO: 1 or 2, or a functional fragment or a functional variant thereof, wherein the functional fragment or the functional variant has an identity of at least 60 % to the nucleic acid sequence according to SEQ ID NO: 1 or 2, wherein the open reading frame (ORF) and the 3'-UTR element are heterologous to each other.

The term "3'-UTR element" refers to a nucleic acid sequence which comprises or consists of a nucleic acid sequence that is derived from a 3'-UTR or from a variant of a 3'-UTR. A "3'-UTR element" preferably refers to a nucleic acid sequence which represents a 3'-UTR of an artificial nucleic acid sequence, such as an artificial mRNA, or which codes for a 3'-UTR of an artificial nucleic acid molecule. Accordingly, in the sense of the present invention, preferably, a 3'-UTR element may be the 3'-UTR of an mRNA, preferably of an artificial mRNA, or it may be the transcription template for a 3'-UTR of an mRNA. Thus, a 3'-UTR element preferably is a nucleic acid sequence which corresponds to the 3'-UTR of an mRNA, preferably to the 3'-UTR of an artificial mRNA, such as an mRNA obtained by transcription of a genetically engineered vector construct. Preferably, a 3'-UTR element in the sense of the present invention functions as a 3'-UTR or codes for a nucleotide sequence that fulfils the function of a 3'-UTR.

The term "a FIG4 gene" generally refers to a gene encoding FIG4, which is also known as, for instance, Sac Domain-Containing Inositol Phosphatase 3, SAC3, S. cerevisiae Homolog of Fig4 (see, for instance, Minagawa et al., 2001. Identification and Characterization of a Sac Domain-containing Phosphoinositide 5-Phosphatase, J. Biol. Chem., Vol. 276, p. 22011-22015; Takasuga and Sasaki, Phosphatidylinositol-3,5-biphosphate: metabolism and physiological functions, Journal of Biochemistry, Vol. 154, No. 3, 2013, p. 211-218). By sequencing clones obtained from a size-fractionated brain cDNA library, Nagase and colleagues cloned human full-length FIG4, which they called KIAA0274 (Nagase et al., 1996. Prediction of the coding sequences of unidentified human genes. VI. The coding sequences of 80 new genes (KIAA0201-KIAA0280) deduced by analysis of cDNA clones from cell line KG-1 and brain. DNA Research 3: 321-329). As used herein, the term "a FIG4 gene" also refers to any FIG4 gene, irrespective of the species, from which it is derived. Specifically, the term refers to a mammalian FIG4 gene. Further, the term "a FIG4 gene" comprises any paralogs and orthologs of a mammalian FIG4 gene. Moreover, any sequence, which is characterized by substantial sequence similarity or identity is referred to as FIG4 gene in the context of the present invention.

Preferably, the at least one open reading frame and the at least one 3'-UTR element are heterologous. The term "heterologous" in this context means that the open reading frame and the 3'-UTR element are not occurring naturally (in nature) in this combination. Preferably, the 3'-UTR element is derived from a different gene than the open reading frame. For example, the ORF may be derived from a different gene than the 3'-UTR element, e.g. encoding a different protein or the same protein but of a different species etc. Preferably, the open reading frame does not code for human FIG4, preferably the open reading frame does not code for FIG4. In specific embodiments it is preferred that the open reading frame does not code for a reporter protein, e.g., selected from the group consisting of globin proteins (particularly beta-globin), luciferase protein, GFP proteins or variants thereof, for example, variants exhibiting at least 70% sequence identity to a globin protein, a luciferase protein, or a GFP protein. In a particularly preferred embodiment, the open reading frame (ORF) does not encode a reporter gene or is not derived from a reporter gene, wherein the reporter gene is preferably not selected from the group consisting of light emitting proteins, preferably not selected from luciferase, fluorescent proteins, preferably not selected from red, blue or green fluorescent proteins; enzymatic reporters; the lacZ gene from E. coli (beta-galactosidase); alkaline phosphatase; secreted embryonic alkaline phosphatase (SEAP); chloramphenicol acetyl transferase (CAT); hormones and cytokines or any derivatives or variants of the afore-mentioned reporter genes. Furthermore, the open reading frame (ORF) does preferably not encode a FIG4 gene or is preferably not derived from a FIG4 gene. It does preferably not encode an eukaryotic FIG4 gene, more preferably not a mammalian FIG4 gene, most preferably not a human FIG4 gene or any variants or derivatives of a FIG4 gene.

Preferably, the at least one 3'-UTR element is functionally linked to the ORF. This means preferably that the

3'-UTR element is associated with the ORF such that it may exert a function, such as a stabilizing function on the expression of the encoded peptide or protein or a stabilizing function on the artificial nucleic acid molecule. Preferably, the ORF and the 3'-UTR element are associated in 5'→3' direction. Thus, preferably, the artificial nucleic acid molecule comprises the structure 5'-ORF-(optional)-linker-3'-UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Further disclosed herein is an artificial nucleic acid molecule, wherein the at least one 3'-UTR element comprises or consists of a nucleic acid sequence which is derived from the 3'-UTR of a vertebrate FIG4 gene or from a variant thereof, preferably from the 3'-UTR of a mammalian FIG4 gene such as e.g. the 3'-UTR of the mouse FIG4 gene, the FIG4 gene of a primate or the human FIG4 gene or from a variant thereof. More preferably the at least one 3'-UTR element comprises or consists of a nucleic acid sequence derived from the 3'-UTR of a primate FIG4 gene, particularly of a human FIG4 gene or from a variant thereof, even more preferably from the 3'-UTR of the human FIG4 gene according to GenBank Accession number NM_014845.5 or from a variant thereof. It is also disclosed herein that the at least one 3'-UTR element comprises or consists of a nucleic acid sequence derived from the 3'-UTR of a non-human primate FIG4 gene, such as a FIG4 homolog from *Macaca mulatta* (NM_001172433), *Callithrix jacchus* (XM_002746998), *Gorilla gorilla gorilla* (XM_004044513), *Pan paniscus* (XM_003805509) or *Nomascus leucogenys* (XM_003255555).

The term "a nucleic acid sequence which is derived from the 3'-UTR of a FIG4 gene" preferably refers to a nucleic acid sequence which is based on the 3'-UTR sequence of a FIG4 gene or on a fragment or part thereof, which is defined above and in the claims. This term includes sequences corresponding to the entire 3'-UTR sequence, i.e. the full length 3'-UTR sequence of a FIG4 gene, and sequences corresponding to a fragment of the 3'-UTR sequence of a FIG4 gene. Preferably, a fragment of a 3'-UTR of a FIG4 gene consists of a continuous stretch of nucleotides corresponding to a continuous stretch of nucleotides in the full-length 3'-UTR of a FIG4 gene, which represents at least 20%, preferably at least 30%, more preferably at least 40%, more preferably at least 50%, even more preferably at least 60%, even more preferably at least 70%, even more preferably at least 80%, and most preferably at least 90% of the full-length 3'-UTR of a FIG4 gene. Such a fragment, in the sense of the present invention, is preferably a functional fragment as described herein. The term "3'-UTR of a FIG4 gene" preferably refers to the 3'-UTR of a naturally occurring FIG4 gene.

The terms "variant of the 3'-UTR of a FIG4 gene" and "variant thereof" in the context of a 3'-UTR of a FIG4 gene refers to a variant of the 3'-UTR of a naturally occurring FIG4 gene, preferably to a variant of the 3'-UTR of a vertebrate FIG4 gene, more preferably to a variant of the 3'-UTR of a mammalian FIG4 gene, even more preferably to a variant of the 3'-UTR of a primate FIG4 gene, in particular a human FIG4 gene as described above. Such variant may be a modified 3'-UTR of a FIG4 gene. For example, a variant 3'-UTR may exhibit one or more nucleotide deletions, insertions, additions and/or substitutions compared to the naturally occurring 3'-UTR from which the variant is derived. Preferably, a variant of a 3'-UTR of a FIG4 gene is at least 40%, preferably at least 50%, more preferably at least 60%, more preferably at least 70%, even more preferably at least 80%, even more preferably at least 90%, most preferably at least 95% identical to the naturally occurring 3'-UTR the variant is derived from. Preferably, the variant is a functional variant as described herein.

The term "a nucleic acid sequence which is derived from a variant of the 3'-UTR of a FIG4 gene" preferably refers to a nucleic acid sequence which is based on a variant of the 3'-UTR sequence of a FIG4 gene or on a fragment or part thereof as described above. This term includes sequences corresponding to the entire sequence of the variant of the 3'-UTR of a FIG4 gene, i.e. the full length variant 3'-UTR sequence of a FIG4 gene, and sequences corresponding to a fragment of the variant 3'-UTR sequence of a FIG4 gene. Preferably, a fragment of a variant of the 3'-UTR of a FIG4 gene consists of a continuous stretch of nucleotides corresponding to a continuous stretch of nucleotides in the full-length variant of the 3'-UTR of a FIG4 gene, which represents at least 20%, preferably at least 30%, more preferably at least 40%, more preferably at least 50%, even more preferably at least 60%, even more preferably at least 70%, even more preferably at least 80%, and most preferably at least 90% of the full-length variant of the 3'-UTR of a FIG4 gene. Such a fragment of a variant, in the sense of the present invention, is preferably a functional fragment of a variant as described herein.

The terms "functional variant", "functional fragment", and "functional fragment of a variant" (also termed "functional variant fragment") in the context of the present invention, mean that the fragment of the 3'-UTR, the variant of the 3'-UTR, or the fragment of a variant of the 3'-UTR of a FIG4 gene fulfils at least one, preferably more than one function of the naturally occurring 3'-UTR of a FIG4 gene of which the variant, the fragment, or the fragment of a variant is derived. Such function may be, for example, stabilizing mRNA and/or stabilizing and/or prolonging protein production from an mRNA and/or increasing protein expression or total protein production from an mRNA, preferably in a mammalian cell, such as in a human cell. It is particularly preferred that the variant, the fragment, and the variant fragment in the context of the present invention fulfil the function of stabilizing an mRNA, preferably in a mammalian cell, such as a human cell, compared to an mRNA comprising a reference 3'-UTR or lacking a 3'-UTR, and/or the function of stabilizing and/or prolonging protein production from an mRNA, preferably in a mammalian cell, such as in a human cell, compared to an mRNA comprising a reference 3'-UTR or lacking a 3'-UTR, and/or the function of increasing protein production from an

mRNA, preferably in a mammalian cell, such as in a human cell, compared to an mRNA comprising a reference 3'-UTR or lacking a 3'-UTR. A reference 3'-UTR may be, for example, a 3'-UTR naturally occurring in combination with the ORF. Furthermore, a functional variant, a functional fragment, or a functional variant fragment of a 3'-UTR of a FIG4 gene preferably does not have a substantially diminishing effect on the efficiency of translation of the mRNA which comprises such variant, fragment, or variant fragment of a 3'-UTR compared to the wild type 3'-UTR from which the variant, the fragment, or the variant fragment is derived. A particularly preferred function of a "functional fragment", a "functional variant" or a "functional fragment of a variant" of the 3'-UTR of a FIG4 gene in the context of the present invention is the stabilization and/or prolongation of protein production by expression of an mRNA carrying the functional fragment, functional variant or functional fragment of a variant as described above.

Preferably, the efficiency of the one or more functions exerted by the functional variant, the functional fragment, or the functional variant fragment, such as mRNA and/or protein production stabilizing efficiency and/or the protein production increasing efficiency, is at increased by at least 5%, more preferably by at least 10%, more preferably by at least 20%, more preferably by at least 30%, more preferably by at least 40%, more preferably by at least 50%, more preferably by at least 60%, even more preferably by at least 70%, even more preferably by at least 80%, most preferably by at least 90% with respect to the mRNA and/or protein production stabilizing efficiency and/or the protein production increasing efficiency exhibited by the naturally occurring 3'-UTR of a FIG4 gene from which the variant, the fragment or the variant fragment is derived.

In the context of the present invention, a fragment of the 3'-UTR of a FIG4 gene or of a variant of the 3'-UTR of a FIG4 gene preferably exhibits a length of at least about 50 nucleotides, preferably of at least about 75 nucleotides, more preferably of at least about 100 nucleotides, even more preferably of at least about 125 nucleotides, most preferably of at least about 150 nucleotides. Preferably, such fragment of the 3'-UTR of a FIG4 gene or of a variant of the 3'-UTR of a FIG4 gene is a functional fragment as described above.

Preferably, the at least one 3'-UTR element of the artificial nucleic acid molecule described herein comprises or consists of a "functional fragment", a "functional variant" or a "functional fragment of a variant" of the 3'-UTR of a FIG4 gene.

Preferably, the at least one 3'-UTR element of the artificial nucleic acid molecule described herein increases the stability of the artificial nucleic acid molecule, e.g. increases the stability of an mRNA as disclosed herein, compared to a respective nucleic acid molecule e.g. an mRNA (reference nucleic acid molecule) lacking a 3'-UTR or comprising a reference 3'-UTR, such as a 3'-UTR naturally occurring in combination with the ORF. The term "respective nucleic acid molecule" or "reference nucleic acid molecule"in this context means that - apart from the different 3'-UTRs - the reference nucleic acid molecule is comparable, preferably identical, to the artificial nucleic acid molecule comprising the 3'-UTR element as disclosed herein.

Preferably, the at least one 3'-UTR element of the artificial nucleic acid molecule described herein increases the stability of protein production from the artificial nucleic acid molecule , e.g. from an mRNA as disclosed herein, compared to a respective mRNA lacking a 3'-UTR or comprising a reference 3'-UTR, such as a 3'-UTR naturally occurring in combination with the ORF. Preferably, the at least one 3'-UTR element of the artificial nucleic acid molecule prolongs protein production from the artificial nucleic acid molecule, e.g. from an mRNA as disclosed herein, compared to a respective mRNA lacking a 3'-UTR or comprising a reference 3'-UTR, such as a 3'-UTR naturally occurring in combination with the ORF. Preferably, the at least one 3'-UTR element of the artificial nucleic acid molecule increases the protein expression and/or total protein production from the artificial nucleic acid molecule, e.g. from an mRNA as disclosed herein, compared to a respective mRNA lacking a 3'-UTR or comprising a reference 3'-UTR, such as a 3'-UTR naturally occurring in combination with the ORF. Preferably, the at least one 3'-UTR element of the artificial nucleic acid molecule does not negatively influence translational efficiency of an mRNA compared to the translational efficiency of a respective mRNA lacking a 3'-UTR or comprising a reference 3'-UTR, such as a 3'-UTR naturally occurring in combination with the ORF. The term "respective mRNA" in this context means that - apart from the different 3'-UTRs - the reference mRNA is comparable, preferably identical, to the mRNA comprising the 3'-UTR element as described herein.

The term "stabilizing and/or prolonging protein production" from an artificial nucleic acid molecule such as an artificial mRNA preferably means that the protein production from the artificial nucleic acid molecule such as the artificial mRNA is stabilized and/or prolonged compared to the protein production from a reference nucleic acid molecule such as a reference mRNA, e.g. comprising a reference 3'-UTR or lacking a 3'-UTR, preferably in a mammalian expression system, such as in HeLa or HDF cells. Thus, protein produced from the artificial nucleic acid molecule such as the artificial mRNA is observable for a longer period of time than what may be seen for a protein produced from a reference nucleic acid molecule. In other words, the amount of protein produced from the artificial nucleic acid molecule such as the artificial mRNA measured over time undercuts a threshold value at a later time point than the amount of protein produced from a reference nucleic acid molecule such as a reference mRNA measured over time. Such a threshold value may be, for example, the amount of protein measured in the initial phase of expression, such as 1, 2, 3, 4, 5 or 6 hours post initiation of expression, such as post transfection of the nucleic acid molecule.

For example, the protein production from the artificial nucleic acid molecule such as the artificial mRNA - in an

amount which is at least the amount observed in the initial phase of expression, such as 1, 2, 3, 4, 5, or 6 hours post initiation of expression, such as post transfection of the nucleic acid molecule - is prolonged by at least about 5 hours, preferably by at least about 10 hours, more preferably by at least about 24 hours compared to the protein production from a reference nucleic acid molecule, such as a reference mRNA, in a mammalian expression system, such as in mammalian cells, e.g. in HeLa or HDF cells. Thus, the artificial nucleic acid molecule according to the present invention preferably allows for prolonged protein production in an amount which is at least the amount observed in the initial phase of expression, such as 1, 2, 3, 4, 5, or 6 hours post initiation of expression, such as post transfection, by at least about 5 hours, preferably by at least about 10 hours, more preferably by at least about 24 hours compared to a reference nucleic acid molecule lacking a 3'-UTR or comprising a reference 3'-UTR.

In preferred embodiments, the period of protein production from the artificial nucleic acid molecule described herein is extended at least 1.5 fold, preferably at least 2 fold, more preferably at least 2.5 fold compared to the protein production from a reference nucleic acid molecule lacking a 3'-UTR or comprising a reference 3'-UTR.

This effect of prolonging protein production may be determined by (i) measuring protein amounts, e.g. obtained by expression of an encoded reporter protein such as luciferase, preferably in a mammalian expression system such as in HeLa or HDF cells, over time, (ii) determining the time point at which the protein amount undercuts the amount of protein observed, e.g., at 1, 2, 3, 4, 5, or 6 hours post initiation of expression, e.g. 1, 2, 3, 4, 5, or 6 hours post transfection of the artificial nucleic acid molecule, and (iii) comparing the time point at which the protein amount undercuts the protein amount observed at 1, 2, 3, 4, 5, or 6 hours post initiation of expression to said time point determined for a nucleic acid molecule lacking a 3'-UTR or comprising a reference 3'-UTR.

Preferably, this stabilizing and/or prolonging effect on protein production is achieved, while the total amount of protein produced from the artificial nucleic acid molecule, e.g. within a time span of 48 or 72 hours, is at least the amount of protein produced from a reference nucleic acid molecule lacking a 3'-UTR or comprising a reference 3'-UTR, such as a 3'-UTR naturally occurring with the ORF of the artificial nucleic acid molecule. Thus, the artificial nucleic acid molecule described herein allows for prolonged and/or stabilized protein production in a mammalian expression system, such as in mammalian cells, e.g. in HeLa or HDF cells, as specified above, wherein the total amount of protein produced from said artificial nucleic acid molecule, e.g. within a time span of 48 or 72 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule.

Thus, "stabilized protein expression" preferably means that there is more uniform protein production from the artificial nucleic acid molecule described herein over a predetermined period of time, such as over 24 hours, more preferably over 48 hours, even more preferably over 72 hours, when compared to a reference nucleic acid molecule, for example, an mRNA comprising a reference 3'-UTR or lacking a 3'-UTR. Accordingly, the level of protein production, e.g. in a mammalian system, from the artificial nucleic acid molecule comprising a 3'-UTR element, e.g. from an mRNA as disclosed herein, preferably does not drop to the extent observed for a reference nucleic acid molecule, such as a reference mRNA as described above. For example, the amount of a protein (encoded by the ORF) observed 6 hours after initiation of expression, e.g. 6 hours post transfection of the artificial nucleic acid molecule according to the present invention into a cell, such as a mammalian cell, may be comparable to the amount of protein observed 48 hours after initiation of expression, e.g. 48 hours post transfection. Thus, the ratio of the amount of protein encoded by the ORF, such as of a reporter protein, e.g., luciferase, observed at 48 hours post initiation of expression, e.g. 48 hours post transfection, to the amount of protein observed 6 hours after initiation of expression, e.g. 6 hours post transfection, is preferably at least about 0.4, more preferably at least about 0.5, more preferably at least about 0.6, even more preferably at least about 0.7. Preferably, the ratio is between about 0.4 and about 4, preferably between about 0.65 and about 3, more preferably between about 0.7 and 2 for a nucleic acid molecule according to the present invention. For a respective reference nucleic acid molecule, e.g. an mRNA comprising a reference 3'-UTR or lacking a 3'-UTR, said ratio may be, e.g. between about 0.05 and about 0.3.

Thus, also disclosed herein is an artificial nucleic acid molecule comprising an ORF and a 3'-UTR element as described above, wherein the ratio of the (reporter) protein amount, e.g. the amount of luciferase, observed 48 hours after initiation of expression to the (reporter) protein amount observed 6 hours after initiation of expression, preferably in a mammalian expression system, such as in mammalian cells, e.g. in HeLa cells, is preferably above about 0.4, more preferably above about 0.5, more preferably above about 0.6, even more preferably above about 0.7, e.g. between about 0.4 and about 4, preferably between about 0.65 and about 3, more preferably between about 0.7 and 2, wherein preferably the total amount of protein produced from said artificial nucleic acid molecule, e.g. within a time span of 48 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule. In a preferred embodiment, the present invention provides an artificial nucleic acid molecule comprising an ORF and a 3'-UTR element as described above, wherein the ratio of the (reporter) protein amount, e.g. the amount of luciferase, observed 72 hours after initiation of expression to the (reporter) protein amount observed 6 hours after initiation of expression, preferably in a mammalian expression system, such as in mammalian cells, e.g. in HeLa cells, is preferably

above about 0.4, more preferably above about 0.5, more preferably above about 0.6, even more preferably above about 0.7, e.g. between about 0.4 and 1.5, preferably between about 0.65 and about 1.15, more preferably between about 0.7 and 1.0, wherein preferably the total amount of protein produced from said artificial nucleic acid molecule, e.g. within a time span of 72 hours, is at least the total amount of protein produced, e.g. within said time span, from a reference nucleic acid molecule lacking a 3'-UTR or comprising a reference 3'-UTR, such as a 3'-UTR naturally occurring with the ORF of the artificial nucleic acid molecule.

"Increased protein expression" in the context of the present invention preferably means an increased protein expression at one time point after initiation of expression compared to the protein expression induced by a reference nucleic acid molecule. Thus, the protein level observed at a certain time point after initiation of expression, e.g. after transfection, of the artificial nucleic acid molecule described herein, e.g. after transfection of an mRNA according to the present invention, for example, 48 or 72 hours post transfection, is preferably higher than the protein level observed at the same time point after initiation of expression, e.g. after transfection, of a reference nucleic acid molecule, such as a reference mRNA comprising a reference 3'-UTR or lacking a 3'-UTR.

"Increased total protein production" from an artificial nucleic acid molecule as disclosed herein refers to an increased protein production over the time span, in which protein is produced from an artificial nucleic acid molecule, preferably in a mammalian expression system, such as in mammalian cells, e.g. in HeLa or HDF cells in comparison to a reference nucleic acid molecule lacking a 3'-UTR or comprising a reference 3'-UTR.

An artificial nucleic acid molecule is disclosed herein, which is characterized by increased expression of the encoded protein in comparison to a reference nucleic acid molecule lacking a 3'-UTR or comprising a reference 3'-UTR, such as a 3'-UTR naturally occurring in connection with the ORF of the artificial nucleic acid molecule, comprising a nucleic acid sequence which is derived from the 3'-UTR of a FIG4 gene or from a variant of the 3'-UTR of a FIG4 gene. In order to assess the *in vivo* protein production by the artificial nucleic acid molecule described herein, the expression of the encoded protein is determined following injection/transfection of the artificial nucleic acid molecule into target cells/tissue and compared to the protein expression induced by the reference nucleic acid molecule. Quantitative methods for determining protein expression are known in the art (e.g. Western-Blot, ELISA, FACS, mass spectometry). Particularly useful in this context is the determination of the expression of reporter proteins like luciferase, Green fluorescent protein (GFP) or secreted alkaline phosphatase (SEAP). Thus, the artificial nucleic acid or a reference nucleic acid molecule is introduced into the target tissue or cell, e.g. via transfection or injection. Several hours or several days (e.g. 6, 12, 24, 48 or 72 hours) post initiation of expression or post introduction of the nucleic acid molecule, a target cell sample is collected and measured via FACS and/or lysed. Afterwards the lysates can be used to detect the expressed protein (and thus determine the efficiency of protein expression) using several methods, e.g. Western-Blot, ELISA, mass spectrometry or by fluorescence or luminescence measurement.

Therefore, if the protein expression from the artificial nucleic acid molecule disclosed herein is compared to the protein expression from a reference nucleic acid molecule at a specific time point (e.g. 6, 12, 24, 48 or 72 hours post initiation of expression or post introduction of the nucleic acid molecule), both nucleic acid molecules are introduced separately into target tissue/cells, a sample from the tissue/cells is collected after a specific time point, protein lysates are prepared according to the particular protocol adjusted to the particular detection method (e.g. Western Blot, ELISA, etc. as known in the art) and the protein is detected by the chosen detection method. As an alternative to the measurement of expressed protein amounts in cell lysates - or, in addition to the measurement of protein amounts in cell lysates prior to lysis of the collected cells - protein amounts may also be determined by using FACS analysis.

If the total amount of protein for a specific time period is to be measured, tissue or cells can be collected after several time points after introduction of the artificial nucleic acid molecule (e.g. 6, 12, 24, 48 and 72 hours post initiation of expression or post introduction of the nucleic acid molecule; usually from different test animals), and the protein amount per time point can be determined as explained above. In order to calculate the cumulative protein amount, a mathematical method of determining the total amount of protein can be used, e.g. the area under the curve (AUC) can be determined according to the following formula:

$$\mathrm{AUC} = \int_a^b f(x)\, d(x)$$

In order to calculate the area under the curve for total amount of protein, the integral of the equation of the expression curve from each end point (a and b) is calculated.

Thus, "total protein production" preferably refers to the area under the curve (AUC) representing protein production over time.

Said increase in stability of the artificial nucleic acid molecule, said increase in stability of protein production, said prolongation of protein production and/or said increase in protein expression and/or total protein production is preferably determined by comparison with a respective reference nucleic acid molecule lacking a 3'-UTR, e.g. an mRNA

lacking a 3'-UTR, or a reference nucleic acid molecule comprising a reference 3'-UTR, such as a 3'-UTR naturally occurring with the ORF as describe above.

The mRNA and/or protein production stabilizing effect and efficiency and/or the protein production increasing effect and efficiency of the variants, fragments and/or variant fragments of the 3'-UTR of a FIG4 gene as well as the mRNA and/or protein production stabilizing effect and efficiency and/or the protein production increasing effect and efficiency of the at least one 3'-UTR element of the artificial nucleic acid molecule described herein may be determined by any method suitable for this purpose known to skilled person. For example, artificial mRNA molecules may be generated comprising a coding sequence/open reading frame (ORF) for a reporter protein, such as luciferase, and no 3'-UTR, a 3'-UTR derived from a naturally occurring FIG4 gene, a 3'-UTR derived from a reference gene (i.e., a reference 3'-UTR, such as a 3'-UTR naturally occurring with the ORF), as 3'-UTR a variant of a 3'-UTR of an FIG4 gene, as 3'-UTR a fragment of a naturally occurring FIG4 gene, or as 3'-UTR a fragment of a variant of a 3'-UTR of a FIG4 gene. Such mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be electroporated into mammalian cells, such as HELA cells, and samples may be analyzed certain time points after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample time points may be determined by quantitative PCR methods. The quantities of reporter protein encoded by the respective mRNAs may be determined, e.g., by Western Blots, ELISA assays, FACS analysis or reporter assays such as luciferase assays depending on the reporter protein used. The effect of stabilizing protein expression and/or prolonging protein expression may be, for example, analyzed by determining the ratio of the protein level observed 48 hours post transfection and the protein level observed 6 hours post transfection. The closer said value is to 1, the more stable the protein expression is within this time period. Such measurements may of course also be performed at 72 or more hours and the ratio of the protein level observed 72 hours post transfection and the protein level observed 6 hours post transfection may be determined to determine stability of protein expression.

The at least one 3'-UTR element of the artificial nucleic acid molecule described herein comprises or consists of a nucleic acid sequence which has an identity of at least about 60%, preferably of at least about 70%, more preferably of at least about 80%, more preferably of at least about 90%, even more preferably of at least about 95%, even more preferably of at least about 99%, most preferably of 100% to the nucleic acid sequence according to SEQ ID No. 1 or SEQ ID No. 2 as shown below, wherein the variants of the sequences (e.g. at least 40% identical) are preferably functional variants as described above:

AAAGAGCGCA GGTCCACCTG GTGGACACGT CTGATTAGCT TAGAACCTGT CTTGTCTCAT
CTTCAAAAGG TAACTTATTA AAAGTCCTTT GCGTCTGAAG CCTTTCTCCT TTTCTGTCAC
TTGCAAATTC CAAATTATAG CTAATAAAGA TGACTAGATA ATTTGC (SEQ ID No. 1)

AAAGAGCGCA GGUCCACCUG GUGGACACGU CUGAUUAGCU UAGAACCUGU CUUGUCUCAU
CUUCAAAAGG UAACUUAUUA AAAGUCCUUU GCGUCUGAAG CCUUUCUCCU UUUCUGUCAC
UUGCAAAUUC CAAAUUAUAG CUAAUAAAGA UGACUAGAUA AUUUGC (SEQ ID No. 2).

The at least one 3'-UTR element of the artificial nucleic acid molecule may also comprise or consist of a fragment of a nucleic acid sequence which has an identity of at least about 60%, preferably of at least about 70%, more preferably of at least about 80%, more preferably of at least about 90%, even more preferably of at least about 95%, even more preferably of at least about 99%, most preferably of 100% to the nucleic acid sequence according to SEQ ID No. 1 or SEQ ID No. 2, wherein the fragment is preferably a functional fragment or a functional variant fragment as described herein and as defined in the claims. Such fragment preferably exhibits a length of at least about 50 nucleotides, preferably of at least about 75 nucleotides, more preferably of at least about 100 nucleotides, even more preferably of at least about 125 nucleotides, most preferably of at least about 150 nucleotides.

Preferably, said variants, fragments or variant fragments are functional variants, functional fragments, or functional variant fragments as described above, exhibiting at least one function of the nucleic acid sequence according to SEQ ID No. 1 or SEQ ID No. 2, such as stabilization of the artificial nucleic acid molecule, stabilizing and/or prolonging protein expression from the artificial nucleic acid molecule, and/or increasing protein production, preferably with an efficiency of at least 40%, more preferably of at least 50%, more preferably of at least 60%, even more preferably of at

least 70%, even more preferably of at least 80%, most preferably of at least 90% of the stabilizing efficiency and/or protein production increasing efficiency exhibited by an artificial nucleic acid molecule comprising the nucleic acid sequence according to SEQ ID No. 1 or SEQ ID NO. 2.

Preferably, the at least one 3'-UTR element, which is preferably of non-viral origin, of the artificial nucleic acid molecule according to the present invention exhibits a length of at least about 50 nucleotides, preferably of at least about 75 nucleotides, more preferably of at least about 100 nucleotides, even more preferably of at least about 125 nucleotides, most preferably of at least about 150 nucleotides. Preferably, the 3'UTR element has a length of less than 500, 400, 300, 250, 200 or less than 150 nucleotides. For example, the 3'-UTR element may exhibit a length of about 50 to about 300 nucleotides, preferably of about 100 to about 250 nucleotides, more preferably of about 150 to about 200 nucleotides.

Furthermore, the artificial nucleic acid molecule according to the present invention may comprise more than one 3'-UTR elements as described above. For example, the artificial nucleic acid molecule described herein may comprise one, two, three, four or more 3'-UTR elements, wherein the individual 3'-UTR elements may be the same or they may be different. For example, the artificial nucleic acid molecule may comprise two essentially identical 3'-UTR elements as described above, e.g. two 3'-UTR elements comprising or consisting of a nucleic acid sequence, which is derived from the 3'-UTR of a FIG4 gene or from a variant of the 3'-UTR of a FIG4 gene, such as a nucleic acid sequence according to SEQ ID No. 1 or 2, functional variants thereof, functional fragments thereof, or functional variant fragments thereof as described above.

Surprisingly, the inventors found that an artificial nucleic acid molecule comprising a 3'-UTR element as described above may represent or may provide an mRNA molecule, which allows for prolonged and/or stabilized protein production. Thus, a 3'-UTR element as described herein may improve stability of protein expression from an mRNA molecule and/or improve translational efficiency.

The artificial nucleic acid molecule described herein may be RNA, such as mRNA, DNA, such as a DNA vector, or may be a modified RNA or DNA molecule. It may be provided as a double-stranded molecule having a sense strand and an anti-sense strand, for example, as a DNA molecule having a sense strand and an anti-sense strand.

The artificial nucleic acid molecule disclosed herein may further comprise optionally a 5'-UTR and/or a 5'-cap. The optional 5'-cap and/or the 5'-UTR are preferably located 5' to the ORF within the artificial nucleic acid molecule.

Preferably, the artificial nucleic acid molecule further comprises a poly(A) sequence and/or a polyadenylation signal. Preferably, the optional poly(A) sequence is located 3' to the at least one 3'-UTR element, preferably the optional poly(A) sequence is connected to the 3'- end of the 3'-UTR element. The connection may be direct or indirect, for example, via a stretch of 2, 4, 6, 8, 10, 20 etc. nucleotides, such as via a linker of 1-50, preferably of 1-20 nucleotides, e.g. comprising or consisting of one or more restriction sites.

In one embodiment, the optional polyadenylation signal is located downstream of the 3'-UTR element. Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. Preferably, the polyadenylation signal, preferably the consensus sequence NNUANA, is located less than about 50 nucleotides, more preferably less than about 30 bases, most preferably less than about 25 bases, for example 21 bases, downstream of the 3'-end of the 3'-UTR element.

Transcription of the artificial nucleic acid molecule, e.g. of an artificial DNA molecule, comprising a polyadenylation signal downstream of the 3'-UTR element will result in a premature-RNA containing the polyadenylation signal downstream of its 3'-UTR element. For example, transcription of a DNA molecule comprising a 3'-UTR element according to SEQ ID No. 1 will result in an RNA having a 3'-UTR element according to the sequence SEQ ID No. 2.

Using an appropriate transcription system will then lead to attachment of a poly(A) sequence to the premature-RNA. For example, the artificial nucleic acid molecule may be a DNA molecule comprising a 3'-UTR element as described above and a polyadenylation signal, which may result in polyadenylation of an RNA upon transcription of this DNA molecule. Accordingly, a resulting RNA may comprise a combination of the 3'-UTR element followed by a poly(A) sequence.

Potential transcription systems are *in vitro* transcription systems or cellular transcription systems etc. Accordingly, transcription of the artificial nucleic acid molecule, e.g. transcription of an artificial nucleic acid molecule comprising an open reading frame, a 3'-UTR element and a polyadenylation signal, may result in an mRNA molecule comprising an open reading frame, a 3'-UTR element and a poly(A) sequence.

Accordingly, the artificial nucleic acid molecule as described herein is preferably an mRNA molecule comprising an open reading frame, a 3'-UTR element as described above and a poly(A) sequence.

In one embodiment, the artificial nucleic acid molecule is an artificial DNA molecule comprising an open reading frame and a sequence according to SEQ ID No. 1 or a sequence having an identity of at least about 40% or more to SEQ ID No. 1 or a fragment thereof as described above. Furthermore, the artificial nucleic acid molecule is preferably an artificial RNA molecule comprising an open reading frame and a sequence according to SEQ ID NO. 2 or a sequence having an identity of at least about 40 % or more to SEQ ID No. 2 or a fragment thereof as described above.

Accordingly, the artificial nucleic acid molecule described herein may serve as a template for an RNA molecule, preferably for an mRNA molecule, which is stabilised and optimized with respect to translation efficiency. In other words, the artificial nucleic acid molecule may be a DNA or RNA which may be used as a template for production of an mRNA. The obtainable mRNA, may, in turn, be translated for production of a desired peptide or protein encoded by the open reading frame. If the artificial nucleic acid molecule is a DNA, it may, for example, be used as a double-stranded storage form for continued and repetitive *in vitro* or *in vivo* production of mRNA.

In one embodiment, the artificial nucleic acid molecule further comprises a poly(A) sequence. For example, a DNA molecule comprising an ORF followed by the FIG4 3' UTR may contain a stretch of thymidine nucleotides which can be transcribed into a poly(A) sequence in the resulting mRNA. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides. Most preferably, the nucleic acid comprises a poly(A) sequence of about 60 to about 70 nucleotides, most preferably 64 adenine nucleotides.

For example, the artificial nucleic acid molecule may comprise a nucleic acid sequence corresponding to the DNA-sequence

```
AAAGAGCGCA GGTCCACCTG GTGGACACGT CTGATTAGCT TAGAACCTGT CTTGTCTCAT
CTTCAAAAGG TAACTTATTA AAAGTCCTTT GCGTCTGAAG CCTTTCTCCT TTTCTGTCAC
TTGCAAATTC CAAATTATAG CTAATAAAGA TGACTAGATA ATTTGCAGAT CTAAAAAAAA
AAAAAAAAAA AAAAAAAAAA AAAAAAAAAA AAAAAAAAAA AAAAAAAAAA AAAAAA
```

(SEQ ID No. 3).

Transcription of such sequences may result in artificial nucleic acid molecules comprising the sequence

```
AAAGAGCGCA GGUCCACCUG GUGGACACGU CUGAUUAGCU UAGAACCUGU CUUGUCUCAU
CUUCAAAAGG UAACUUAUUA AAAGUCCUUU GCGUCUGAAG CCUUUCUCCU UUUCUGUCAC
UUGCAAAUUC CAAAUUAUAG CUAAUAAAGA UGACUAGAUA AUUUGCAGAU CUAAAAAAAA
AAAAAAAAAA AAAAAAAAAA AAAAAAAAAA AAAAAAAAAA AAAAAAAAAA AAAAAA
```

(SEQ ID No. 4).

Such artificial RNA molecules, i.e. artificial nucleic acid molecules comprising a sequence according to SEQ ID No. 4 may also be obtainable *in vitro* by common methods of chemical-synthesis without being necessarily transcribed from a DNA progenitor.

In a particularly preferred embodiment, the artificial nucleic acid molecule is an RNA molecule, preferably an mRNA molecule comprising in 5'-to-3'-direction an open reading frame, a 3'-UTR element as described above and a poly(A) sequence.

In a preferred embodiment, the open reading frame does not code for FIG4, particularly not for mammalian FIG4, provided that the 3'-UTR element is identical to the 3'-UTR of a mammalian FIG4 gene. In some further preferred embodiments, the open reading frame does not code for human FIG4 according to GenBank Accession number NM_014845.5, provided that the 3'-UTR element is identical to the 3'-UTR of human FIG4. In some further preferred embodiments, the open reading frame does not code for FIG4 or variants thereof, provided that the 3'-UTR element is a sequence which is identical to SEQ ID No. 1 or SEQ ID No. 2.

In one embodiment, the artificial DNA molecule comprises an open reading frame, preferably an open reading frame which encodes a peptide or protein other than FIG4; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 1; and a polyadenylation signal and/or a poly(A) sequence. Furthermore, the artificial DNA molecule as described herein comprises an open reading frame, preferably an open reading frame which encodes any peptide or protein other than FIG4; a 3'-UTR element comprising or consisting of a sequence, which has at least about 60%, preferably at least about 70%, more preferably at least about 80%, more preferably at least about 90%, even more preferably at least about 95%; even more preferably at least 99%; even more preferably 100% sequence identity to SEQ ID No. 3.

Furthermore, the disclosure relates to an artificial RNA molecule, preferably an artificial mRNA molecule, an artificial viral RNA molecule, or an artificial self-replicating RNA molecule (replicon), comprising an open reading frame, preferably an open reading frame which encodes a peptide or protein other than FIG4; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 2; and a polyadenylation signal and/or a poly(A) sequence. Furthermore, the disclosure relates to an artificial RNA molecule, preferably an artificial mRNA molecule or an artificial viral RNA molecule, comprising an open reading frame, preferably an open reading frame which encodes a peptide or protein other than FIG4; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to SEQ ID No. 4.

The artificial nucleic acid molecule, preferably an artificial mRNA, as described herein may be characterized by enhanced stability and prolonged expression of the encoded peptide or protein. Without being bound by any theory, enhanced stability of protein expression and thus prolonged protein expression may result from reduction in degradation of the artificial nucleic acid molecule, such as an artificial mRNA molecule. Accordingly, the 3'-UTR element may prevent the artificial nucleic acid from degradation and decay.

In some embodiments, the artificial nucleic acid molecule may comprise a histone stem-loop in addition to the nucleic acid sequence derived from the 3'-UTR of a FIG4 gene. Such artificial nucleic acid molecule according to the present invention, for example, may comprise in 5'-to-3'-direction an ORF, an 3'-UTR element, an optional histone stem-loop sequence, an optional poly(A) sequence/or polyadenylation signal and an optional poly(C) sequence. It may also comprise in 5'-to-3'-direction an ORF, an 3'-UTR element, an optional poly(A) sequence, an optional poly(C) sequence and an optional histone stem-loop sequence.

In a preferred embodiment, the artificial nucleic acid molecule comprises at least one histone stem-loop sequence.

Such histone stem-loop sequences are preferably selected from histone stem-loop sequences as disclosed in WO 2012/019780.

A histone stem-loop sequence, suitable to be used within the present invention, is preferably selected from at least one of the following formulae (I) or (II):

formula (I) (stem-loop sequence without stem bordering elements):

$$\underbrace{[N_{0-2}GN_{3-5}]}_{\text{stem1}}\;\underbrace{[N_{0-4}(U/T)N_{0-4}]}_{\text{loop}}\;\underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\\text{bordering element}}}\;\underbrace{[N_{0-2}GN_{3-5}]}_{\text{stem1}}\;\underbrace{[N_{0-4}(U/T)N_{0-4}]}_{\text{loop}}\;\underbrace{[N_{3-5}CN_{0-2}]}_{\text{stem2}}\;\underbrace{N_{1-6}}_{\substack{\text{stem2}\\\text{bordering element}}}$$

wherein:

stem1 or stem2 bordering elements $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;

stem1 $[N_{0-2}GN_{3-5}]$ is reverse complementary or partially reverse complementary with element stem2, and is a consecutive sequence between of 5 to 7 nucle-

otides;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;

loop sequence $[N_{0-4}(U/T)N_{0-4}]$ is located between elements stem1 and stem2, and is a consecutive sequence of 3 to 5 nucleotides, more preferably of 4 nucleotides;
wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;

stem2 $[N_{3-5}CN_{0-2}]$ is reverse complementary or partially reverse complementary with element stem1, and is a consecutive sequence between of 5 to 7 nucleotides;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;
wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and
wherein C is cytidine or an analogue thereof, and may be optionally replaced by a guanosine or an analogue thereof provided that its complementary nucleoside guanosine in stem1 is replaced by cytidine;

wherein

stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

According to a further preferred embodiment the histone stem-loop sequence may be selected according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\substack{\text{stem1}\\ \text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\ \text{bordering element}}}$$

wherein:

N, C, G, T and U are as defined above.

According to a further more particularly preferred embodiment of the first aspect, the artificial nucleic acid molecule sequence may comprise at least one histone stem-loop sequence according to at least one of the following specific formulae (Ib) or (IIb):

formula (Ib) (stem-loop sequence without stem bordering elements):

$$\underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}$$

formula (IIb) (stem-loop sequence with stem bordering elements):

$$\underbrace{N_{4-5}}_{\substack{\text{stem1}\\ \text{bordering element}}}\ \underbrace{[N_{1}GN_{4}]}_{\text{stem1}}\ \underbrace{[N_{2}(U/T)N_{1}]}_{\text{loop}}\ \underbrace{[N_{4}CN_{1}]}_{\text{stem2}}\ \underbrace{N_{4-5}}_{\substack{\text{stem2}\\ \text{bordering element}}}$$

wherein:

N, C, G, T and U are as defined above.

A particular preferred histone stem-loop sequence is the sequence according to SEQ ID NO: 5: CAAAGGCTCTTTTCAGAGCCACCA or more preferably the corresponding RNA sequence of the nucleic acid sequence according to SEQ ID NO: 5.

As an example, the single elements may be present in the artificial nucleic acid molecule in the following order:

5'-cap - 5'-UTR - ORF - 3'-UTR element - histone stem-loop - poly(A)/(C) sequence;
5'-cap - 5'-UTR - ORF - 3'-UTR element - poly(A)/(C) sequence - histone stem-loop;
5'-cap - 5'-UTR - ORF - IRES - ORF - 3'-UTR element - histone stem-loop - poly(A)/(C) sequence;
5'-cap - 5'-UTR - ORF - IRES - ORF - 3'-UTR element - histone stem-loop - poly(A)/(C) sequence - poly(A)/(C) sequence;
5'-cap - 5'-UTR - ORF - IRES - ORF - 3'-UTR element - poly(A)/(C) sequence - histone stem-loop;
5'-cap - 5'-UTR - ORF - IRES - ORF - 3'-UTR element - poly(A)/(C) sequence - poly(A)/(C) sequence - histone stem-loop;
5'-cap - 5'-UTR - ORF - 3'-UTR element - poly(A)/(C) sequence - poly(A)/(C) sequence;
5'-cap - 5'-UTR - ORF - 3'-UTR element - poly(A)/(C) sequence - poly(A)/(C) sequence - histone stem loop; etc.

In some embodiments, the artificial nucleic acid molecule comprises further elements such as a 5'-cap, a poly(C) sequence and/or an IRES-motif. A 5'-cap may be added during transcription or post-transcriptionally to the 5'end of an

RNA. Furthermore, the artificial nucleic acid molecule, particularly if the nucleic acid is in the form of an mRNA or codes for an mRNA, may be modified by a sequence of at least 10 cytidines, preferably at least 20 cytidines, more preferably at least 30 cytidines (so-called "poly(C) sequence"). In particular, the artificial nucleic acid molecule may contain, especially if the nucleic acid is in the form of an (m)RNA or codes for an mRNA, a poly(C) sequence of typically about 10 to 200 cytidine nucleotides, preferably about 10 to 100 cytidine nucleotides, more preferably about 10 to 70 cytidine nucleotides or even more preferably about 20 to 50 or even 20 to 30 cytidine nucleotides. Most preferably, the nucleic acid comprises a poly(C) sequence of 30 cytidine residues. Thus, preferably the artificial nucleic acid molecule comprises, preferably in 5'-to-3' direction, an ORF, at least one 3'-UTR element as described above, a poly(A) sequence or a polyadenylation signal, and a poly(C) sequence.

An internal ribosome entry site (IRES) sequence or IRES-motif may separate several open reading frames, for example if the artificial nucleic acid molecule encodes for two or more peptides or proteins. An IRES-sequence may be particularly helpful if the artificial nucleic acid molecule is a bi- or multicistronic nucleic acid molecule.

Furthermore, the artificial nucleic acid molecule may comprise additional 5'-elements, preferably a 5'-UTR, a promoter, or a 5'-UTR and a promoter containing-sequence. The promoter may drive and or regulate transcription of the artificial nucleic acid molecule, for example of an artificial DNA-molecule as disclosed herein. Furthermore, the 5'-UTR may consist or may comprise the 5'-UTR of a gene as defined herein. Furthermore the 5'-UTR may interact with the 3'-UTR element and thus may support the stabilising effect of the 3'-UTR element. Such elements may further support stability and translational efficiency. Accordingly, in some embodiments, the artificial nucleic acid molecule, preferably an mRNA-molecule, comprises in 5'-to-3'-direction at least one of the following structures

5'-cap - 5'-UTR - ORF - 3'-UTR element - histone stem-loop - poly(A)/(C) sequence;
5'-cap - 5'-UTR - ORF - 3'-UTR element - poly(A)/(C) sequence - histone stem-loop;
5'-cap - 5'-UTR - ORF - IRES - ORF - 3'-UTR element - histone stem-loop - poly(A)/(C) sequence;
5'-cap - 5'-UTR - ORF - IRES - ORF - 3'-UTR element - histone stem-loop - poly(A)/(C) sequence - poly(A)/(C) sequence;
5'-cap - 5'-UTR - ORF - IRES - ORF - 3'-UTR element - poly(A)/(C) sequence - histone stem-loop;
5'-cap - 5'-UTR - ORF - IRES - ORF - 3'-UTR element - poly(A)/(C) sequence - poly(A)/(C) sequence - histone stem-loop;
5'-cap - 5'-UTR - ORF - 3'-UTR element - poly(A)/(C) sequence - poly(A)/(C) sequence;
5'-cap - 5'-UTR - ORF - 3'-UTR element - poly(A)/(C) sequence - poly(A)/(C) sequence - histone stem loop.

In a particularly preferred embodiment of the present invention the artificial nucleic acid molecule comprises at least one 5'-untranslated region element (5'UTR element) which comprises or consists of a nucleic acid sequence which is derived from the 5'UTR of a TOP gene or which is derived from a fragment, homolog or variant of the 5'UTR of a TOP gene.

It is particularly preferred that the 5'UTR element does not comprise a TOP-motif or a 5'TOP, as defined above. The nucleic acid sequence which is derived from the 5'UTR of a TOP gene is derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'UTR element is preferably selected from 5'-UTR elements comprising or consisting of a nucleic acid sequence which is derived from a nucleic acid sequence selected from the group consisting of SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID NOs. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID NOs. 1-1363, SEQ ID NO. 1395, SEQ ID NO. 1421 and SEQ ID NO. 1422 of the patent application WO2013/143700.

In a preferred embodiment, the 5'UTR element comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363,

SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence.

In a particularly preferred embodiment, the 5'UTR element comprises or consists of a nucleic acid sequence which is preferably derived from a 5'UTR of a TOP gene encoding a ribosomal protein or from a variant of a 5'UTR of a TOP gene encoding a ribosomal protein. For example, the 5'UTR element comprises or consists of a nucleic acid sequence which is derived from a 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence which is derived from a 5'UTR of a TOP gene encoding a ribosomal Large protein (RPL) or from a homolog or variant of a 5'UTR of a TOP gene encoding a ribosomal Large protein (RPL). For example, the 5'UTR element comprises or consists of a nucleic acid sequence which is derived from a 5'UTR of a nucleic acid sequence according to any of SEQ ID NOs: SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In a particularly preferred embodiment, the 5'UTR element comprises or consists of a nucleic acid sequence which is derived from the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'UTR element does not comprise the 5'TOP of said gene.

Accordingly, in a particularly preferred embodiment, the 5'UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 6 (5'-UTR of human ribosomal protein Large 32 lacking the 5' terminal oligopyrimidine tract: GGCGCTGCCTACGGAGGTGGCAGCCATCTCCTTCTCGGCATC; corresponding to SEQ ID No. 1368 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 6 or more preferably to a corresponding RNA sequence, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the artificial nucleic acid molecule comprises a 5'UTR element which comprises or consists of a nucleic acid sequence which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a homolog or variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8,

9 or 10 upstream of the start codon (A(U/T)G) of the gene it is derived from.

Preferably, the artificial nucleic acid molecule described herein, preferably the open reading frame, is at least partially G/C modified. Thus, the artificial nucleic acid molecule may be thermodynamically stabilized by modifying the G (guanosine)/C (cytidine) content of the molecule. The G/C content of the open reading frame of the artificial nucleic acid molecule may be increased compared to the G/C content of the open reading frame of a corresponding wild type sequence, preferably by using the degeneration of the genetic code. Thus, the encoded amino acid sequence of the artificial nucleic acid molecule is preferably not modified by the G/C modification compared to the coded amino acid sequence of the particular wild type sequence. The codons of the coding sequence or the whole artificial nucleic acid molecule, e.g. an mRNA, may therefore be varied compared to the wild type coding sequence, such that they include an increased amount of G/C nucleotides while the translated amino acid sequence is maintained. Due to the fact that several codons code for one and the same amino acid (so-called degeneration of the genetic code), it is feasible to alter codons while not altering the encoded peptide/protein sequence (so-called alternative codon usage). Hence, it is possible to specifically introduce certain codons (in exchange for the respective wild-type codons encoding the same amino acid), which are more favourable with respect to stability of RNA and/or with respect to codon usage in a subject (so-called codon optimization).

Depending on the amino acid to be encoded by the coding region of the artificial nucleic acid molecule as defined herein, there are various possibilities for modification of the nucleic acid sequence, e.g. the open reading frame, compared to its wild type coding region. In the case of amino acids, which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present. In contrast, codons which contain A and/or U/T nucleotides may be modified by substitution of other codons which code for the same amino acids but contain no A and/or U/T. For example, the codons for Pro can be modified from CC(U/T) or CCA to CCC or CCG; the codons for Arg can be modified from CG(U/T) or CGA or AGA or AGG to CGC or CGG; the codons for Ala can be modified from GC(U/T) or GCA to GCC or GCG; the codons for Gly can be modified from GG(U/T) or GGA to GGC or GGG. In other cases, although A or (U/T) nucleotides cannot be eliminated from the codons, it is however possible to decrease the A and (U/T) content by using codons which contain a lower content of A and/or (U/T) nucleotides. Examples of these are: The codons for Phe can be modified from (U/T)(U/T)(U/T) to (U/T) (U/T)C; the codons for Leu can be modified from (U/T) (U/T)A, (U/T) (U/T)G, C(U/T) (U/T) or C(U/T)A to C(U/T)C or C(U/T)G; the codons for Ser can be modified from (U/T)C(U/T) or (U/T)CA or AG(UAT) to (U/T)CC, (U/T)CG or AGC; the codon for Tyr can be modified from (U/T)A(U/T) to (U/T)AC; the codon for Cys can be modified from (U/T)G(U/T) to (U/T)GC; the codon for His can be modified from CA(U/T) to CAC; the codon for Gln can be modified from CAA to CAG; the codons for lie can be modified from A(U/T)(U/T) or A(U/T)A to A(U/T)C; the codons for Thr can be modified from AC(U/T) or ACA to ACC or ACG; the codon for Asn can be modified from AA(U/T) to AAC; the codon for Lys can be modified from AAA to AAG; the codons for Val can be modified from G(U/T)(U/T) or G(U/T)A to G(U/T)C or G(U/T)G; the codon for Asp can be modified from GA(UAT) to GAC; the codon for Glu can be modified from GAA to GAG; the stop codon (U/T)AA can be modified to (U/T)AG or (U/T)GA. In the case of the codons for Met (A(U/T)G) and Trp ((U/T)GG), on the other hand, there is no possibility of sequence modification without altering the encoded amino acid sequence. The substitutions listed above can be used either individually or in all possible combinations to increase the G/C content of the open reading frame of the artificial nucleic acid molecule as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the artificial nucleic acid molecule as defined herein is increased by at least 7%, more preferably by at least 15%, particularly preferably by at least 20%, compared to the G/C content of the wild type coding region without altering the encoded amino acid sequence, i.e. using the degeneracy of the genetic code. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the artificial nucleic acid molecule or a fragment, variant or derivative thereof are substituted, thereby increasing the G/C content of said open reading frame.

In this context, it is particularly preferable to increase the G/C content of the open reading frame of the artificial nucleic acid molecule as defined herein, to the maximum (i.e. 100% of the substitutable codons), compared to the wild type open reading frame, without altering the encoded amino acid sequence.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the artificial nucleic acid molecule as defined herein, to an increased extent, the translation of the corresponding modified nucleic acid sequence is less efficient than in the case where codons coding for relatively "frequent" tRNAs are present.

Thus, the open reading frame of the artificial nucleic acid molecule is preferably modified compared to the corresponding wild type coding region such that at least one codon of the wild type sequence which codes for a tRNA

which is relatively rare in the cell is exchanged for a codon which codes for a tRNA which is comparably frequent in the cell and carries the same amino acid as the relatively rare tRNA. By this modification, the open reading frame of the artificial nucleic acid molecule as defined herein, is modified such that codons for which frequently occurring tRNAs are available may replace codons which correspond to rare tRNAs. In other words, according to the invention, by such a modification all codons of the wild type open reading frame which code for a rare tRNA may be exchanged for a codon which codes for a tRNA which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the artificial nucleic acid molecule is to be expressed, preferably with respect to the system in which the artificial nucleic acid molecule is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* degradation by an exo- or endonuclease, and/or for further improving stability of protein expression from the artificial nucleic acid moleculedescribed herein, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule according to the present invention is not significantly impaired by said modifications.

Generally, the artificial nucleic acid molecule may comprise any native (= naturally occurring) nucleotide, e.g. guanosine, uracil, adenosine, and/or cytosine or an analogue thereof. In this respect, nucleotide analogues are defined as natively and non-natively occurring variants of the naturally occurring nucleotides adenosine, cytosine, thymidine, guanosine and uridine. Accordingly, analogues are e.g. chemically derivatized nucleotides with non-natively occurring functional groups, which are preferably added to or deleted from the naturally occurring nucleotide or which substitute the naturally occurring functional groups of a nucleotide. Accordingly, each component of the naturally occurring nucleotide may be modified, namely the base component, the sugar (ribose) component and/or the phosphate component forming the backbone (see above) of the RNA sequence. Analogues of guanosine, uridine, adenosine, thymidine and cytosine include, without implying any limitation, any natively occurring or non-natively occurring guanosine, uridine, adenosine, thymidine or cytosine that has been altered e.g. chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyl-adenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 2-thio-cytosine, 3-methyl-cytosine, 4-acetyl-cytosine, 4-Thiouridine, 5-(carboxyhydroxymethyl)-uracil, 5,6-Dihydrouridine, 5-Aminoallylcytidine, 5-Aminoallyl-deoxy-uridine, 5-Bromouridine, 5-carboxymehtylaminomethyl-2-thio-uracil, 5-carboxymethylamonomethyl-uracil, 5-Chloro-Ara-cytosine, 5-Fluoro-uridine, 5-Iodouridine, 5-methoxycarbonylmethyl-uridine, 5-methoxy-uridine, 5-methyl-2-thio-uridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromo-guanosine, 8-Mercapto-guanosine, 8-Oxoguanosine, Benzimidazole-riboside, Beta-D-mannosyl-queosine, Dihydro-uracil, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given according to certain embodiments of the invention to those analogues that increase the protein expression of the encoded peptide or protein or that increase the immunogenicity of the artificial nucleic acid molecule and/or do not interfere with a further modification of the artificial nucleic acid molecule that has been introduced.

According to a particular embodiment, the artificial nucleic acid molecule can contain a lipid modification.

In a particularly preferred embodiment, the artificial nucleic acid molecule may further comprise one or more of the modifications described in the following:

Chemical modifications:

The term "modification" as used herein with regard to the artificial nucleic acid molecule may refer to chemical

modifications comprising backbone modifications as well as sugar modifications or base modifications.

In this context, the artificial nucleic acid molecule, preferably an RNA molecule, as defined herein may contain nucleotide analogues/modifications, e.g. backbone modifications, sugar modifications or base modifications. A backbone modification in connection with the present invention is a modification, in which phosphates of the backbone of the nucleotides contained in a nucleic acid molecule as defined herein are chemically modified. A sugar modification in connection with the present invention is a chemical modification of the sugar of the nucleotides of the nucleic acid molecule as defined herein. Furthermore, a base modification in connection with the present invention is a chemical modification of the base moiety of the nucleotides of the nucleic acid molecule of the nucleic acid molecule. In this context, nucleotide analogues or modifications are preferably selected from nucleotide analogues which are applicable for transcription and/or translation.

Sugar Modifications:

The modified nucleosides and nucleotides, which may be incorporated into the artificial nucleic acid molecule, preferably an RNA, as described herein, can be modified in the sugar moiety. For example, the 2' hydroxyl group (OH) of an RNA molecule can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), $-O(CH_2CH_2O)_nCH_2CH_2OR$; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. $NH_2$; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified nucleic acid molecule can include nucleotides containing, for instance, arabinose as the sugar.

Backbone Modifications:

The phosphate backbone may further be modified in the modified nucleosides and nucleotides, which may be incorporated into the artificial nucleic acid molecule, preferably an RNA, as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

Base Modifications:

The modified nucleosides and nucleotides, which may be incorporated into the artificial nucleic acid molecule, preferably an RNA molecule, as described herein, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-triphosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio- 1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine .

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl) adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.

In specific embodiments, a modified nucleoside is 5'-O-(1-Thiophosphate)-Adenosine, 5'-O-(1-Thiophosphate)-Cytidine, 5'-O-(1-Thiophosphate)-Guanosine, 5'-O-(1-Thiophosphate)-Uridine or 5'-O-(1-Thiophosphate)-Pseudouridine.

In further specific embodiments the artificial nucleic acid molecule, preferably an RNA molecule, may comprise nucleoside modifications selected from 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxy-thymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, alpha-thio-adenosine, 8-azido-adenosine, 7-deaza-adenosine.

Lipid modification:

According to a further embodiment, the artificial nucleic acid molecule, preferably an RNA, as defined herein can contain a lipid modification. Such a lipid-modified RNA typically comprises an RNA as defined herein. Such a lipid-modified RNA molecule as defined herein typically further comprises at least one linker covalently linked with that RNA molecule, and at least one lipid covalently linked with the respective linker. Alternatively, the lipid-modified RNA molecule comprises at least one RNAmolecule as defined herein and at least one (bifunctional) lipid covalently linked (without a linker) with that RNA molecule. According to a third alternative, the lipid-modified RNA molecule comprises an artificial nucleic acid molecule, preferably an RNA molecule, as defined herein, at least one linker covalently linked with that RNA molecule, and at least one lipid covalently linked with the respective linker, and also at least one (bifunctional) lipid covalently linked (without a linker) with that RNA molecule. In this context, it is particularly preferred that the lipid modification is present at the terminal ends of a linear RNA sequence.

Modification of the 5'-end of the modified RNA:

According to another preferred embodiment of the invention, the artificial nucleic acid molecule, preferably an

RNA molecule, as defined herein, can be modified by the addition of a so-called "5' CAP" structure.

A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. m7GpppN is the 5'-CAP structure which naturally occurs in mRNA transcribed by polymerase II and is therefore not considered as modification comprised in the modified RNA according to the invention. This means the artificial nucleic acid molecule, preferably an RNA molecule, may comprise a m7GpppN as 5'-CAP, but additionally the artificial nucleic acid molecule, preferably an RNA molecule, comprises at least one further modification as defined herein.

Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures are regarded as at least one modification comprised in the artificial nucleic acid molecule, preferably in an RNA molecule.

Particularly preferred modified 5'-CAP structures are CAP1 (methylation of the ribose of the adjacent nucleotide of m7G), CAP2 (methylation of the ribose of the 2nd nucleotide downstream of the m7G), CAP3 (methylation of the ribose of the 3rd nucleotide downstream of the m7G), CAP4 (methylation of the ribose of the 4th nucleotide downstream of the m7G), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In a preferred embodiment, the artificial nucleic acid molecule comprises, preferably from 5' to 3' direction, the following elements:

a 5'-UTR;
at least one open reading frame (ORF), wherein the ORF preferably comprises at least one modification with respect to the wildtype sequence;
a 3'-UTR derived from the 3'-UTR of a FIG4 homolog, preferably of human FIG4;
a poly(A) sequence, preferably comprising 64 adenylates;
a poly(C) sequence, preferably comprising 30 cytidylates;
a histone stem-loop sequence.

In another preferred embodiment, the artificial nucleic acid molecule comprises or consists of a nucleotide sequence as shown according to SEQ ID NO: 7 (see Fig. 3) or the complementary DNA sequence.

In a preferred embodiment, the at least one open reading frame encodes a therapeutic protein or peptide. In another embodiment, an antigen is encoded by the at least one open reading frame, such as a pathogenic antigen, a tumour antigen, an allergenic antigen or an autoimmune antigen. Therein, the administration of the artificial nucleic acid molecule encoding the antigen is used in a genetic vaccination approach against a disease involving said antigen.

In an alternative embodiment, an antibody is encoded by the at least one open reading frame of the artificial nucleic acid molecule.

Antigens:

Pathogenic antigens:

The artificial nucleic acid molecule described herein may encode a protein or a peptide, which comprises a pathogenic antigen or a fragment, variant or derivative thereof. Such pathogenic antigens are derived from pathogenic organisms, in particular bacterial, viral or protozoological (multicellular) pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi,

Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

Tumour antigens:

In a further embodiment the artificial nucleic acid molecule described herein may encode a protein or a peptide, which comprises a peptide or protein comprising a tumour antigen, a fragment, variant or derivative of said tumour antigen, preferably, wherein the tumour antigen is a melanocyte-specific antigen, a cancer-testis antigen or a tumour-specific antigen, preferably a CT-X antigen, a non-X CT-antigen, a binding partner for a CT-X antigen or a binding partner for a non-X CT-antigen or a tumour-specific antigen, more preferably a CT-X antigen, a binding partner for a non-X CT-antigen or a tumour-specific antigen or a fragment, variant or derivative of said tumour antigen; and wherein each of the nucleic acid sequences encodes a different peptide or protein; and wherein at least one of the nucleic acid sequences encodes for 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin idiotype of a lymphoid blood cell or a T cell receptor idiotype of a lymphoid blood cell, or a fragment, variant or derivative of said tumour antigen; preferably survivin or a homologue thereof, an antigen from the MAGE-family or a binding partner thereof or a fragment, variant or derivative of said tumour antigen. Particularly preferred in this context are the tumour antigens NY-ESO-1, 5T4, MAGE-C1, MAGE-C2, Survivin, Muc-1, PSA, PSMA, PSCA, STEAP and PAP.

In a preferred embodiment, the artificial nucleic acid molecule encodes a protein or a peptide, which comprises a therapeutic protein or a fragment, variant or derivative thereof.

Therapeutic proteins as defined herein are peptides or proteins, which are beneficial for the treatment of any inherited or acquired disease or which improves the condition of an individual. Particularly, therapeutic proteins play an important role in the creation of therapeutic agents that could modify and repair genetic errors, destroy cancer cells or pathogen infected cells, treat immune system disorders, treat metabolic or endocrine disorders, among other functions. For instance, Erythropoietin (EPO), a protein hormone can be utilized in treating patients with erythrocyte deficiency, which is a common cause of kidney complications. Furthermore adjuvant proteins, therapeutic antibodies are encompassed by therapeutic proteins and also hormone replacement therapy which is e.g. used in the therapy of women in menopause. In more recent approaches, somatic cells of a patient are used to reprogram them into pluripotent stem cells, which replace the disputed stem cell therapy. Also these proteins used for reprogramming of somatic cells or used for differentiating of stem cells are defined herein as therapeutic proteins. Furthermore, therapeutic proteins may be used for other purposes, e.g. wound healing, tissue regeneration, angiogenesis, etc. Furthermore, antigen-specific T-cell receptors, antigen-specific B cell receptors and fragments and variants thereof are defined herein as therapeutic proteins.

Therefore therapeutic proteins can be used for various purposes including treatment of various diseases like e.g. infectious diseases, neoplasms (e.g. cancer or tumour diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

In this context, particularly preferred therapeutic proteins which can be used inter alia in the treatment of metabolic or endocrine disorders are selected from: Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (Iduronate-2-sulphatase) (Mucopolysaccharidosis II (Hunter syndrome)), Integrins αVβ3, αVβ5 and α5β1 (Bind matrix macromolecules and proteinases, angiogenesis), Iuduronate sulfatase (Hunter syndrome), Laronidase (Hurler and Hurler-Scheie forms of mucopolysaccharidosis I), N-acetylgalactosamine-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF, Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta 1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF (TGF-alpha, TGF-beta (TGFbeta1, TGFbeta2, and TGFbeta3))) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F und PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome, Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumors), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing). The terms in brackets refer to the particular disease for which the therapeutic protein is used in the treatment. These and other proteins are understood to be therapeutic, as they are meant to treat the subject by replacing its defective endogenous production of a functional protein in sufficient amounts. Accordingly, such therapeutic proteins are typically mammalian, in particular human proteins.

For the treatment of blood disorders, diseases of the circulatory system, diseases of the respiratory system, cancer or tumour diseases, infectious diseases or immunedeficiencies following therapeutic proteins may be used: Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of

arteriovenous cannula), Tenecteplase (Acute myocardial infarction), Urokinase (Pulmonary embolism), Angiostatin (Cancer), Anti-CD22 immunotoxin (Relapsed CD33+ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxy-ribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhoea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when refractory to TNF-alpha inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin alpha1 (Hepatitis B and C). (in brackets is the particular disease for which the therapeutic protein is used in the treatment)

Also described herein is a vector comprising

a. an open reading frame (ORF) and/or a cloning site, e.g. for insertion of an open reading frame or a sequence comprising an open reading frame; and

b. at least one 3'-untranslated region element (3'-UTR element) comprising a nucleic acid sequence which is derived from the 3'-UTR of a FIG4 gene or from a variant of the 3'-UTR of a FIG4 gene.

The at least one 3'-UTR element and the ORF are as described above for the artificial nucleic acid molecule. The cloning site may be any sequence that is suitable for introducing an open reading frame or a sequence comprising an open reading frame, such as one or more restriction sites. Thus, the vector comprising a cloning site is preferably suitable for inserting an open reading frame into the vector, preferably for inserting an open reading frame 5' to the 3'-UTR element. Preferably the cloning site or the ORF is located 5' to the 3'-UTR element, preferably in close proximity to the 5'-end of the 3'-UTR element. For example, the cloning site or the ORF may be directly connected to the 5'-end of the 3'-UTR element or they may be connected via a stretch of nucleotides, such as by a stretch of 2, 4, 6, 8, 10, 20 etc. nucleotides as described above for the artificial nucleic acid molecule.

Preferably, the vector disclosed herein is suitable for producing the artificial nucleic acid molecule described herein, preferably for producing an artificial mRNA as described herein, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognized by a polymerase, such as by an RNA polymerase, e.g. by a eukaryotic, prokaryotic, viral, or phage RNA polymerase. The vector preferably comprises a phage RNA polymerase promoter such as an SP6, T3 or T7, more preferably a T7 promoter. Preferably, the vector is suitable for *in vitro* transcription using a phage based *in vitro* transcription system, such as a T7 RNA polymerase based *in vitro* transcription system.

The vector may be used directly for expression of the encoded peptide or protein in cells or tissue. For this purpose the vector comprises particular elements which are necessary for expression in that cells/tissue, e.g. particular promoter

sequences as a CMV promoter.

The vector may further comprise a poly(A) sequence and/or a polyadenylation signal as described above for the artificial nucleic acid molecule.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector.

In a preferred embodiment, the vector described herein comprises the artificial nucleic acid molecule as disclosed herein.

Preferably, a DNA vector as described herein comprises a sequence according to SEQ ID No. 1, SEQ ID No. 3, a sequence complementary to SEQ ID No. 7 or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% sequence identity to the nucleic acid sequence according to SEQ ID No. 1, SEQ ID No. 3, SEQ ID No. 7 or a fragment thereof as described above, preferably a functional fragment thereof.

Preferably, an RNA vector as described herein comprises a sequence according to SEQ ID No. 2, SEQ ID No. 4, SEQ ID No. 7 or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% sequence identity to the nucleic acid sequence according to SEQ ID No. 2, SEQ ID No. 4, SEQ ID No. 7 or a fragment thereof, preferably a functional fragment thereof.

Preferably, the vector is a circular molecule. Preferably, the vector is a double-stranded molecule, such as a double-stranded DNA molecule. Such circular, preferably double stranded DNA molecule may be used conveniently as a storage form for the artificial nucleic acid molecule. Furthermore, it may be used for transfection of cells, for example, cultured cells. Also it may be used for *in vitro* transcription for obtaining the artificial RNA molecule as disclosed herein.

Preferably, the vector, more preferably the circular vector, is linearizable, for example, by restriction enzyme digestion. The vector preferably comprises a cleavage site, such as a restriction site, preferably a unique cleavage site, located 3' to the 3'-UTR element, or - if present - located 3' to the poly(A) sequence or polyadenylation signal, or - if present - located 3' to the poly(C) sequence, or - if present - located 3' to the histone stem-loop. Thus, preferably, the product obtained by linearizing the vector terminates at the 3'-end or several nucleotides 3' of the 3'-end of the 3'-UTR element, or - if present - of the poly(A) sequence or polyadenylation signal, or - if present - of the poly(C) sequence, or - if present -of the histone stem-loop. If the vector comprises the artificial nucleic acid molecule described herein, a restriction site, preferably a unique restriction site, is preferably located 3' to the 3'-end of the artificial nucleic acid molecule.

Also disclosed herein is a cell comprising the artificial nucleic acid molecule or the vector described herein. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the artificial nucleic acid molecule or the vector and/or translating the open reading frame of the artificial nucleic acid molecule or the vector. For example, the cell may be used for recombinant protein production.

The cells disclosed herein are, for example, obtainable by standard nucleic acid transfer methods, such as standard transfection, transduction or transformation methods. For example, the artificial nucleic acid molecule or the vector described herein may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of human subject, a domestic animal, a laboratory animal, such as a mouse or rat cell. Preferably the cell is a human cell. The cell may be a cell of an established cell line, such as a CHO, BHK, 293T, COS-7, HELA, HEK, etc. or the cell may be a primary cell, such as a human dermal fibroblast (HDF) cell etc., preferably a cell isolated from an organism. The cell is preferably an isolated cell of a mammalian subject, more preferably of a human subject. For example, the cell may be an immune cell, such as a dendritic cell, a cancer or tumor cell, or any somatic cell etc., preferably of a mammalian subject, preferably of a human subject.

In one aspect, the present invention provides a pharmaceutical composition comprising the artificial nucleic acid molecule as described herein, the vector as described herein, or the cell as described herein. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable vehicles, diluents and/or excipients and/or one or more adjuvants. In the context of the present invention, a pharmaceutically acceptable vehicle typically includes a liquid or non-liquid basis for the inventive pharmaceutical composition. In one

embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with the artificial nucleic acid, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the inventive pharmaceutical composition may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component which may be suitable for depot and delivery of an artificial nucleic acid molecule or vector. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, proline-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (I):

$$\{(Arg)_l;(Lys)_m;(His)_n;(Orn)_o;(Xaa)_x\}; \qquad \text{formula (I)}$$

wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (I)) as shown above and which comprise or are additionally modified to comprise at least one -SH moeity, may be, without being restricted thereto, selected from subformula (Ia):

$$\{(Arg)_l;(Lys)_m;(His)_n;(Orn)_o;(Xaa')_x(Cys)_y\} \qquad \text{subformula (Ia)}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (Ib):

$$Cys_1 \{(Arg)_l;(Lys)_m;(His)_n;(Orn)_o;(Xaa)_x\} Cys_2 \qquad \text{subformula (Ib)}$$

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$ and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

According to another embodiment, the pharmaceutical composition according to the invention may comprise an adjuvant in order to enhance the immunostimulatory properties of the pharmaceutical composition. In this context, an adjuvant may be understood as any compound, which is suitable to support administration and delivery of the components such as the artificial nucleic acid molecule or vector comprised in the pharmaceutical composition according to the invention. Furthermore, such an adjuvant may, without being bound thereto, initiate or increase an immune response of the innate immune system, i.e. a non-specific immune response. With other words, when administered, the pharmaceutical composition according to the invention typically initiates an adaptive immune response directed to the antigen encoded by the artificial nucleic acid molecule. Additionally, the pharmaceutical composition according to the invention may generate an (supportive) innate immune response due to addition of an adjuvant as defined herein to the pharmaceutical composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMER™ (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE™ (propanediamine); BAY R1005™ ((N-(2-deoxy-2-L-leucylamino-b-D-glucopyranosyl)-N-octadecyl-dodecanoylamide hydroacetate); CALCITRIOL™ (1-alpha,25-dihydroxy-vitamin D3); calcium phosphate gel; CAP™ (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c] quinoline-4-amine); ImmTher™ (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMS™; ISCOPREP 7.0.3. ™; liposomes; LOXORIBINE™ (7-allyl-8-oxoguanosine); LT oral adjuvant (E.coli labile enterotoxin-protoxin); microspheres and microparticles of any composition;

MF59™; (squalene-water emulsion); MONTANIDE ISA 51™ (purified incomplete Freund's adjuvant); MONTANIDE ISA 720™ (metabolisable oil adjuvant); MPL™ (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE™ (Nac-Mur-L-Ala-D-Gln-OCH3); MURAPALMITINE™ and D-MURAPALMITINE™ (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURAN™ (beta-glucan); PLGA, PGA and PLA (homo- and co-polymers of lactic acid and glycolic acid; microspheres/nanospheres); PLURONIC L121™; PMMA (polymethyl methacrylate); PODDS™ (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON™ (QS-21); Quil-A (Quil-A saponin); S-28463 (4-amino-otec-dimethyl-2-ethoxymethyl-1H-imidazo[4,5 c]quinoline-1-ethanol); SAF-1™ ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Ala-dipalmitoxypropylamide); Theronyl-MDP (Termurtide™ or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the artificial nucleic acid molecule or the vector of the pharmaceutical composition with the cationic or polycationic compound. Association or complexing the artificial nucleic acid molecule or the vector of the pharmaceutical composition with cationic or polycationic compounds as defined herein preferably provides adjuvant properties and confers a stabilizing effect to the artificial nucleic acid molecule or the vector of the pharmaceutical composition. Particularly such preferred, such cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, pIsl, FGF, Lactoferrin, Transportan, Buforin-2, Bac715-24, SynB, SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: 1-(2,3-sioleyloxy)propyl) -N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as -aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the artificial nucleic acid molecule or the vector, preferably an RNA, of the composition, may be selected from following proteins or peptides having the following total formula (I): (Arg)l;(Lys)m;(His)n;(Orn)o;(Xaa)x, wherein l +

m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

The ratio of the artificial nucleic acid or the vector to the cationic or polycationic compound may be calculated on the basis of the nitrogen/phosphate ratio (N/P-ratio) of the entire nucleic acid complex. For example, 1 μg RNA typically contains about 3 nmol phosphate residues, provided the RNA exhibits a statistical distribution of bases. Additionally, 1 μg peptide typically contains about x nmol nitrogen residues, dependent on the molecular weight and the number of basic amino acids. When exemplarily calculated for (Arg)9 (molecular weight 1424 g/mol, 9 nitrogen atoms), 1 μg (Arg)9 contains about 700 pmol (Arg)9 and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 μg RNA, 6 nmol phosphate are to be calulated for the RNA; 1 μg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of nucleic acid:peptide in the complex, and most preferably in the range of about 0.7-1.5.

Patent application WO2010/037539 describes an immunostimulatory composition and methods for the preparation of an immunostimulatory composition. Accordingly, in a preferred embodiment of the invention, the composition is obtained in two separate steps in order to obtain both, an efficient immunostimulatory effect and efficient translation of the artificial nucleic acid molecule or vector described herein. Therein, a so called "adjuvant component" is prepared by complexing - in a first step - the artificial nucleic acid molecule or vector, preferably an RNA, of the adjuvant component with a cationic or polycationic compound in a specific ratio to form a stable complex. In this context, it is important, that no free cationic or polycationic compound or only a neglibly small amount remains in the adjuvant component after complexing the nucleic acid. Accordingly, the ratio of the nucleic acid and the cationic or polycationic compound in the adjuvant component is typically selected in a range that the nucleic acid is entirely complexed and no free cationic or polycationic compound or only a neclectably small amount remains in the composition. Preferably the ratio of the adjuvant component, i.e. the ratio of the nucleic acid to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the artificial nucleic acid molecule or vector, preferably an RNA molecule, is added in a second step to the complexed nucleic acid molecule, preferably an RNA, of the adjuvant component in order to form the (immunostimulatory) composition of the invention. Therein, the artificial nucleic acid molecule or vector, preferably an RNA, of the invention is added as free nucleic acid, i.e. nucleic acid, which is not complexed by other compounds. Prior to addition, the free artificial nucleic acid molecule or vector is not complexed and will preferably not undergo any detectable or significant complexation reaction upon the addition of the adjuvant component.

Suitable adjuvants may furthermore be selected from nucleic acids having the formula (II): GlXmGn, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof.

Other suitable adjuvants may furthermore be selected from nucleic acids having the formula (III): ClXmCn, wherein: C is cytosine, uracil or an analogue of cytosine or uracil; X is guanosine, uracil, adenosine, thymidine, cytosine or an analogue of the above-mentioned nucleotides; l is an integer from 1 to 40, wherein when l = 1 C is cytosine or an analogue thereof, when l > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

The pharmaceutical composition according to the present invention preferably comprises a "safe and effective amount" of the components of the pharmaceutical composition, particularly of the artificial nucleic acid molecule, the vector and/or the cells as defined herein. As used herein, a "safe and effective amount" means an amount sufficient to significantly induce a positive modification of a disease or disorder as defined herein. At the same time, however, a "safe

and effective amount" preferably avoids serious side-effects and permits a sensible relationship between advantage and risk. The determination of these limits typically lies within the scope of sensible medical judgment.

In a further aspect, the present invention provides the artificial nucleic acid molecule, the vector, the cell as described herein or the pharmaceutical composition according to the present invention for use as a medicament, for example, as vaccine (in genetic vaccination) or in gene therapy.

The artificial nucleic acid molecule, the vector, the cell as described herein, or the pharmaceutical composition according to the present invention are particularly suitable for any medical application which makes use of the therapeutic action or effect of peptides, polypeptides or proteins, or where supplementation of a particular peptide or protein is needed. Thus, the present invention provides the artificial nucleic acid molecule, the vector, the cell as described herein, or the pharmaceutical composition according to the present invention for use in the treatment or prevention of diseases or disorders amenable to treatment by the therapeutic action or effect of peptides, polypeptides or proteins or amenable to treatment by supplementation of a particular peptide, polypeptide or protein. For example, the artificial nucleic acid molecule, the vector, the cell as described herein, or the pharmaceutical composition according to the present invention may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments which benefit from a stable and prolonged presence of therapeutic peptides, polypeptides or proteins in a subject to be treated are especially suitable as medical application in the context of the present invention, since the 3'-UTR element provides for a stable and prolonged expression of the encoded peptide or protein of the artificial nucleic acid molecule or vector. Thus, a particularly suitable medical application for the artificial nucleic acid molecule, the vector, the cell as described herein, or the pharmaceutical composition according to the present invention is vaccination. Thus, the present invention provides the artificial nucleic acid molecule the vector the cell as described herein, or the pharmaceutical composition according to the present invention for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, the artificial nucleic acid molecule or the vector described herein comprises an ORF encoding an amino acid sequence comprising or consisting of an antigen or immunogen, e.g. an epitope of a pathogen or a tumour-associated antigen, a 3'-UTR element as described above, and optional further components, such as a poly(A) sequence etc.

In the context of medical application, in particular, in the context of vaccination, it is preferred that the artificial nucleic acid molecule is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector according to the present invention, e.g., in the context of gene therapeutic treatments, it may be desirable that the artificial nucleic acid molecule or the vector is a DNA molecule.

The artificial nucleic acid molecule, the vector, the cell as described herein, or the pharmaceutical composition according to the present invention may be administered orally, parenterally, by inhalation spray, topically, rectally, nasally, buccally, vaginally, via an implanted reservoir or via jet injection. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. The artificial nucleic acid molecule, the vector, the cell as described herein, or the pharmaceutical composition according to the present invention is preferably administered via needle-free injection (e.g. jet injection).

Preferably, the artificial nucleic acid molecule, the vector, the cell as described herein, or the pharmaceutical composition according to the present invention is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents. Preferably, the solutions or suspensions are administered via needle-free injection (e.g. jet injection).

The artificial nucleic acid molecule, the vector, the cell as described herein, or the pharmaceutical composition according to the present invention may also be administered orally in any orally acceptable dosage form including, but not limited to, capsules, tablets, aqueous suspensions or solutions.

The artificial nucleic acid molecule, the vector, the cell as described herein, or the pharmaceutical composition according to the present invention may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule, the vector, the cell as described herein, or the pharmaceutical composition according to the present invention may be formulated in a suitable ointment suspended or dissolved in one or more carriers.

In one embodiment, the use as a medicament comprises the step of transfection of mammalian cells, preferably *in vitro* or *ex vivo* transfection of mammalian cells, more preferably *in vitro* transfection of isolated cells of a subject to be treated by the medicament. If the use comprises the *in vitro* transfection of isolated cells, the use as a medicament may further comprise the readministration of the transfected cells to the patient. The use of the artificial nucleic acid molecules or the vector as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present invention also provides a method for treating or preventing a disease or disorder as described above comprising administering the artificial nucleic acid molecule, the vector, the cell as described herein, or the pharmaceutical composition according to the present invention to a subject in need thereof.

Furthermore, the artificial nucleic acid molecule or the vector as described herein are provided for use in a method for treating or preventing a disease or disorder comprising transfection of a cell with the artificial nucleic acid molecule or with the vector. Said transfection may be performed *in vitro, ex vivo* or *in vivo.* Transfection of a cell is preferably performed *in vitro* and the transfected cell is administered to a subject in need thereof, preferably to a human patient. Preferably, the cell which is to be transfected *in vitro* is an isolated cell of the subject, preferably of the human patient. Thus, the artificial nucleic acid molecule or the vector as described herein are provided for use in a method of treatment comprising the steps of isolating a cell from a subject, preferably from a human patient, transfecting the isolated cell with the artificial nucleic acid or the vector, and administering the transfected cell to the subject, preferably the human patient.

The method of treating or preventing a disorder described herein is preferably a vaccination method or a gene therapy method as described above.

As described above, the 3'-UTR element is capable of stabilizing an mRNA molecule and/or of stabilizing and/or prolonging the protein production from an mRNA molecule. Thus, in a further aspect, the present invention relates to an in vitro method for stabilizing an RNA molecule, preferably an mRNA molecule, comprising the step of associating the RNA molecule, preferably the mRNA molecule, or a vector encoding the RNA molecule, with a 3'-UTR element comprising or consisting of a nucleic acid sequence which is derived from the 3'-UTR of a FIG4 gene or from a variant of the 3'-UTR of an FIG4 gene, preferably with the 3'-UTR element as described herein with respect to the artificial nucleic acid molecule of the pharmaceutical composition according to the invention.

Furthermore, the present invention relates to an in vitro method for stabilizing and/or prolonging protein production from an artificial nucleic acid molecule or from a vector, preferably from an mRNA molecule, and/or for stabilizing and/or prolonging protein production from an artificial nucleic acid molecule or from a vector, preferably from an mRNA molecule, the method comprising the step of associating the artificial nucleic acid molecule or the vector, preferably the mRNA molecule, with a 3'-UTR element which comprises or consists of a nucleic acid sequence which is derived from the 3'-UTR of a FIG4 gene or from a variant of the 3'-UTR of a FIG4 gene and which is as described herein with respect to the artificial nucleic acid molecule of the pharmaceutical composition according to the invention.

The term "associating the artificial nucleic acid molecule or the vector with a 3'-UTR element" in the context of the present invention preferably means functionally associating or functionally combining the artificial nucleic acid molecule or the vector with the 3'-UTR element. This means that the artificial nucleic acid molecule or the vector and the 3'-UTR element, preferably the 3'-UTR element as described above, are associated or coupled such that the function of the 3'-UTR element, e.g., the RNA and/or protein production stabilizing function, is exerted. Typically, this means that the 3'-UTR element is integrated into the artificial nucleic acid molecule or the vector, preferably the mRNA molecule, 3' to an open reading frame, preferably immediately 3' to an open reading frame, preferably between the open reading frame and a poly(A) sequence or a polyadenylation signal. Preferably, the 3'-UTR element is integrated into the artificial nucleic acid molecule or the vector, preferably the mRNA, as 3'-UTR, i.e. such that the 3'-UTR element is the 3'-UTR of the artificial nucleic acid molecule or the vector, preferably the mRNA, i.e., such that it extends from the 3'-side of the open reading frame to the 5'-side of a poly(A) sequence or a polyadenylation signal, optionally connected via a short linker, such as a sequence comprising or consisting of one or more restriction sites. Thus, preferably, the term "associating

the artificial nucleic acid molecule or the vector with a 3'-UTR element" means functionally associating the 3'-UTR element with an open reading frame located within the artificial nucleic acid molecule or the vector, preferably within the mRNA molecule. The 3'-UTR and the ORF are as described above for the artificial nucleic acid molecule, for example, preferably the ORF and the 3'-UTR are heterologous, e.g. derived from different genes, as described above.

In a further aspect, the present invention provides the use of a 3'-UTR element, preferably the 3'-UTR element as described above, for increasing the stability of an RNA molecule, preferably of an mRNA molecule, wherein the 3'-UTR element comprises or consists of a nucleic acid sequence, which is derived from the 3'-UTR of a FIG4 gene or from a variant of the 3'-UTR of a FIG4 gene.

Furthermore, the present invention provides the use of a 3'-UTR element, preferably the 3'-UTR element as described above, for increasing protein production from an artificial nucleic acid molecule or a vector, preferably from an mRNA molecule, and/or for stabilizing and/or prolonging protein production from an artificial nucleic acid molecule or a vector molecule, preferably from an mRNA molecule, wherein the 3'-UTR element comprises or consists of a nucleic acid sequence which is derived from the 3'-UTR of a FIG4 gene or from a variant of the 3'-UTR of a FIG4 gene as described above.

The uses according to the present invention preferably comprise associating the artificial nucleic acid molecule, the vector, or the RNA with the 3'-UTR element as described above.

The compounds and ingredients of the inventive pharmaceutical composition may also be manufactured and traded separately of each other. Thus, the invention relates further to a kit or kit of parts comprising an artificial nucleic acid molecule, a vector, a cell as described herein, and/or a pharmaceutical composition according to the invention. Preferably, such kit or kits of parts may, additionally, comprise instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

The following Figures, Sequences and Examples are intended to illustrate the invention further. They are not intended to limit the subject matter of the invention thereto.

Fig. 1: Effect of the human FIG4 3'-UTR on luciferase expression from an artificial mRNA. Therein, the mRNA comprising the human FIG4 3'-UTR is an mRNA according to the present invention. It comprises - in 5'-to-3'-direction - a 32L4 5'-UTR5'-UTR, a GC-enriched sequence encoding *Photinus pyralis* luciferase, a 3'-UTR element according to SEQ ID No. 2, a poly(A) sequence having a length of 64 adenines and a poly(C) sequence of 30 cytidine nucleotides. A markedly extended protein expression from the artificial mRNA containing the human FIG4 3'-UTR corresponding to SEQ ID No. 2 is observable compared to the mRNA lacking the FIG4 3' UTR. Data are graphed as RLU ± SD (mean of relative light units ± standard deviation) for triplicate transfections. RLU are summarized in Example 5.1.
The following abbreviations are used:

- PpLuc (GC): GC-enriched mRNA sequence coding for *Photinus pyralis* luciferase
- A64: poly(A)-sequence with 64 adenylates
- C30: poly(C)-sequence with 30 cytidylates
- hSL: a histone stem-loop sequence taken from (Cakmakci, Lerner, Wagner, Zheng, & William F Marzluff, 2008. Mol. Cell. Biol. 28(3):1182-94).
- 32L4: 5'-UTR of human ribosomal protein Large 32 lacking the 5' terminal oligopyrimidine tract
- fig4: 3'-UTR of human fig4 (Homo sapiens FIG4 homolog, SAC1 lipid phosphatase domain containing (S. cerevisiae) (FIG4), mRNA).

Fig. 2: mRNA sequence of 32L4 - PpLuc(GC) - A64 - C30 - hSL (SEQ ID NO: 8). The 5'-UTR is derived from human ribosomal protein Large 32 mRNA lacking the 5' terminal oligopyrimidine tract. The PpLuc(GC) ORF is highlighted in italics.

Fig. 3: mRNA sequence of 32L4 - PpLuc(GC) - fig4-A64-C30-hSL (SEQ ID NO: 7). The 3'-UTR is derived from human Fig4 transcript. The PpLuc(GC) ORF is highlighted in italics, the 3' UTR is underlined.

Fig. 4: DNA sequence corresponding to mRNA PpLuc(GC)-A64-C30-hSL (SEQ ID NO: 9). The PpLuc(GC) ORF is highlighted in italics.

Fig. 5: mRNA sequence of PpLuc(GC)-fig4-A64-C30-hSL (SEQ ID NO: 10). The 3'-UTR is derived from human Fig4 transcript. The PpLuc(GC) ORF is highlighted in italics, the 3' UTR is underlined.

Fig. 6: Effect of the presence of a fig4 3'-UTR on protein expression from an mRNA. Luciferase expression is shown after transfection of HeLa cells with an mRNA comprising a fig4 3'-UTR and the respective control construct without a fig4 3'-UTR.

Fig. 7: Protein expression after intradermal injection in mice. Luciferase expression has been measured after injection of an mRNA comprising a fig4 3'-UTR or an mRNA comprising an albumin7 3'-UTR, respectively. A. Time course of protein expression. B. Area under the curve (AUC).

Fig. 8: DNA sequence corresponding to mRNA RPL32 - PpLuc(GC) - albumin7 - A64 - C30 - histoneSL (SEQ ID NO: 11).

Examples

**1. Preparation of DNA-templates**

A vector for *in vitro* transcription was used containing a T7 promoter followed by a 32L4 5'-UTR, a GC-enriched sequence coding for *Photinus pyralis* luciferase (Ppluc(GC)), and an A64 poly(A) sequence. The A64 poly(A) sequence is followed by C30, a histone stem-loop sequence and a restriction site used for linearization of the vector before *in vitro* transcription.
This vector was modified to include the 3'-UTR of the human fig4 transcript 3' of the PpLuc ORF.
mRNAs obtained from these vectors by *in vitro* transcription are designated as:

32L4-PpLuc(GC)-A64-C30-hSL (Fig. 2; SEQ ID NO: 8)
32L4-PpLuc(GC)-fig4-A64-C30-hSL (Fig. 3; SEQ ID NO: 7)

A further vector for *in vitro* transcription was used containing a T7 promoter followed by a GC-enriched sequence encoding *Photinus pyralis* luciferase (Ppluc(GC)), and an A64 poly(A) sequence. The A64 poly(A) sequence is followed by C30, a histone stem-loop sequence and a restriction site used for linearization of the vector before *in vitro* transcription. Also this vector was modified to include the 3'UTR of the human fig4 transcript 3' of the PpLuc ORF. mRNAs obtained from these vectors by in vitro transcription are designated as:

PpLuc(GC)-A64-C30-hSL (Fig. 4; SEQ ID NO: 9)
PpLuc(GC)-fig4-A64-C30-hSL (Fig. 5; SEQ ID NO: 10).

**2. *In vitro* transcription**

The DNA-template according to Example 1 was linearized and transcribed *in vitro* using T7 RNA polymerase. The DNA template was then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

**3. Luciferase expression by mRNA lipofection**

Human HeLa cells were seeded in 96 well plates at a density of $1x10^4$ cells per well medium (RPMI 1640 medium with L-glutamine and 25mM Hepes (Lonza, Basel, Switzerland) to which 10% FCS, 1% Pen/Strep, 1%Glutamine were added). The following day, cells were washed in Opti-MEM® I Reduced Serum Medium (Gibco, Life Technologies, Carlsbad, CA, USA) and then transfected with 12,5 ng per well of Lipofectamine2000-complexed PpLuc-encoding mRNA in Opti-MEM. Untransfected cells served as control. mRNA coding for *Renilla reniformis* luciferase (RrLuc) was transfected together with PpLuc mRNA to control for transfection efficiency (1 ng of RrLuc mRNA per well). 90 minutes after start of transfection, Opti-MEM was exchanged for medium. 24, 48, 72 hours after transfection, medium was aspirated and cells were lysed in 100 µl of Passive Lysis buffer (Promega). Lysates were stored at -80°C until luciferase activity was measured.

**4. Luciferase measurement**

Luciferase activity was measured as relative light units (RLU) in a Hidex Chameleon plate reader. The activities of Ppluc and Rrluc are measured sequentially from a single sample in a dual luciferase assay. The PpLuc activity was measured first at 2 seconds measuring time using 20 µl of lysate and 50 µl of Beetle juice (pjk GmbH). After 1500ms

delay RrLuc activity is measured with 50μl Renilla juice (pjk GmbH).

**5. Luciferase expression by intradermal mRNA injection**

Anaesthetized, female Balb/C mice received 4 intradermal injections per mouse (10 intradermal injections per group). Per injection 2μg of PpLuc enconding mRNA were administered in 40μl of 80% RiLa. 1 day, 2 days, 3 days, 4 days and 7 days after injection the anaesthetized mice are injected intraperitoneally with 150μl of Luciferin solution (20g/l). 10 minutes after Luciferin injection PpLuc levels are measured by optical imaging using the IVIS Lumina II System.

## RESULTS

**6.1 Protein expression from mRNA containing a Fig4 3'-UTR is prolonged.**

To investigate the effect of Fig4 3'-UTR protein expression from mRNA, an mRNA containing FIG4 3'-UTR (Fig. 3; SEQ ID NO: 7) was compared to a corresponding mRNA lacking the FIG4' 3'-UTR (Fig. 2; SEQ ID NO: 8).

Human HeLa cells were transfected with Luciferase encoding mRNAs and Luciferase levels were measured 24, 48, and 72 hours after transfection. The PpLuc signal was corrected for transfection efficiency by the signal of cotransfected RrLuc (see following Table 1 and Fig. 1).

**Table 1:**

| PpLuc expression normalized to RrLuc (mean RLU values are given) | | | |
|---|---|---|---|
| **mRNA** | **RLU at 24 hours** | **RLU at 48 hours** | **RLU at 72 hours** |
| 32L-PpLuc(GC)-A64-C30-hSL | 2104506 | 254095 | 23803 |
| 32 L-PpLuc(GC)-FIG4-A64-C30-hSL | 2194153 | 840772 | 290597 |

Luciferase was clearly expressed from mRNA lacking the FIG4 3'-UTR. The FIG4 3'-UTR significantly extended luciferase expression.

In the same manner, the expression of luciferase expressed from PpLuc(GC)-A64-C30-hSL (SEQ ID NO: 9; Fig. 4) and the expression of luciferase expressed from PpLuc(GC)-fig4-A64-C30-hSL (SEQ ID NO: 10; Fig. 5). The results are shown in Table 2 and Fig. 6.

**Table 2**

| **mRNA** | **RLU at 24 hours** | **RLU at 48 hours** | **RLU at 72 hours** |
|---|---|---|---|
| PpLuc(GC)-A64-C30-hSL | 499469 | 85598 | 13542 |
| PpLuc(GC)-fig4-C30-hSL | 505983 | 238912 | 69598 |

Also in the case of SEQ ID NO: 10, the FIG4 3'-UTR significantly extended luciferase expression after transfection in HeLa cells.

**6.2 Protein expression from mRNA containing a Fig4 3'UTR is increased after injection in mice.**

To investigate the effect of the Fig4 3'UTR on protein expression from mRNA, an mRNA containing the fig4 3'UTR (32L4-PpLuc(GC)-fig4-A64-C30-hSL; SEQ ID NO: 7; Fig. 3) was compared to an mRNA containing the albumin7 3'UTR (RPL32 - PpLuc(GC) - albumin7 - A64 - C30 - histoneSL; SEQ ID NO: 11; Fig. 8), the latter of which has been reported to extend the expression of a protein encoded by an associated ORF (see WO2013/143698). Mice were injected intradermally with luciferase encoding the respective mRNAs. 1 day, 2 days, 3 days, 4 days and 7 days after injection, luciferase levels were measured. The results are shown in Table 3 and Fig. 7.

**Table 3:**

| PpLuc expression (mean RLU values are given) | | |
|---|---|---|
| | **32L4-PpLuc(GC)-albumin7-A64-C30-hSL** | **32 L4-PpLuc(GC)-fig4-A64-C3 0-hSL** |
| Day 1 | 176720000 | 329600000 |

(continued)

| PpLuc expression (mean RLU values are given) | | |
|---|---|---|
| | **32L4-PpLuc(GC)-albumin7-A64-C30-hSL** | **32 L4-PpLuc(GC)-fig4-A64-C3 0-hSL** |
| Day 2 | 89590000 | 183050000 |
| Day 3 | 32020000 | 51960000 |
| Day 4 | 12082000 | 36150000 |
| Day 7 | 1203400 | 2749800 |

In comparison with the albumin7 3'-UTR, the fig4 3'-UTR significantly increased the expression of luciferase protein from the respective mRNA.

## Claims

1. A pharmaceutical composition comprising an artificial nucleic acid molecule comprising

    a. at least one open reading frame (ORF); and
    b. at least one 3'-untranslated region element (3'-UTR element) comprising a nucleic acid sequence according to SEQ ID NO: 1 or 2, or a functional fragment or a functional variant thereof, wherein the functional fragment or the functional variant has an identity of at least 60% to the nucleic acid sequence according to SEQ ID NO: 1 or 2,

    wherein the open reading frame (ORF) and the 3'-UTR element are heterologous to each other.

2. The pharmaceutical composition according to claim 1, wherein the open reading frame (ORF) does not encode a reporter gene or is not derived from a reporter gene, wherein the reporter gene is preferably not selected from the group consisting of light emitting proteins, preferably not luciferase, fluorescent proteins, preferably not red, blue or green fluorescent proteins; enzymatic reporters; the lacZ gene from *E. coli* (beta-galactosidase); alkaline phosphatase; secreted embryonic alkaline phosphatase (SEAP); chloramphenicol acetyl transferase (CAT); hormones and cytokines.

3. The pharmaceutical composition according to claim 1 or 2, wherein the open reading frame (ORF) does not encode a FIG4 gene or is not derived from a FIG4 gene, preferably not an eukaryotic FIG4 gene, more preferably not a mammalian FIG4 gene, most preferably not a human FIG4 gene.

4. The pharmaceutical composition according to any one of claims 1 to 3, wherein the at least one 3'-UTR element stabilizes/prolongs protein production from said artificial nucleic acid molecule.

5. The pharmaceutical composition according to any one of claims 1-4, wherein the at least one 3'-UTR element comprises or consists of a nucleic acid sequence, which has an identity of at least about 70%, preferably of at least about 80%, more preferably of at least about 90%, even more preferably of at least about 95%, even more preferably of at least about 99% to the nucleic acid sequence according to SEQ ID No. 1 or SEQ ID No. 2, or wherein the at least one 3'-UTR element comprises or consists of a fragment of a nucleic acid sequence, which has an identity of at least about 70%, preferably of at least about 80%, more preferably of at least about 90%, even more preferably of at least about 95%, even more preferably of at least about 99% to the nucleic acid sequence according to SEQ ID No. 1 or SEQ ID No. 2.

6. The pharmaceutical composition according to any one of claims 1-5, wherein the artificial nucleic acid molecule further comprises:
    c. a poly(A) sequence and/or a polyadenylation signal, wherein the poly(A) sequence or the polyadenylation signal is preferably located 3' of the 3'-UTR element.

7. The pharmaceutical composition according to any one of claims 1-6, wherein the artificial nucleic acid molecule further comprises a 5'-cap structure, a poly(C) sequence, a histone stem-loop, and/or an IRES-motif.

**8.** The pharmaceutical composition according to any one of claims 1-7, wherein the nucleic acid comprises an additional 5'-element, preferably a 5'-UTR, a promoter, or a 5'-UTR and a promoter-containing sequence.

**9.** The pharmaceutical composition according to claim 8, wherein the 5'-UTR is a 5'-TOP UTR.

**10.** The pharmaceutical composition according to any one of claims 1-9, wherein the artificial nucleic acid molecule, preferably the open reading frame, is at least partially G/C modified, preferably wherein the G/C content of the open reading frame is increased compared to the wild type open reading frame, and/or wherein the open reading frame comprises a codon-optimized region, preferably, wherein the open reading frame is codon-optimized.

**11.** The pharmaceutical composition according to any one of claims 1-10, wherein the artificial nucleic acid molecule is an RNA, preferably an mRNA molecule.

**12.** The pharmaceutical composition comprising a vector comprising the artificial nucleic acid molecule as defined by any one of claims 1-11.

**13.** The pharmaceutical composition according to claim 12, wherein the vector is a plasmid vector or a viral vector, preferably a plasmid vector.

**14.** The pharmaceutical composition according to any one of claims 1 to 13 comprising a cell comprising the artificial nucleic acid molecule as defined by any one of claims 1-11 or comprising the vector as defined by claim 12 or 13.

**15.** The pharmaceutical composition according to any one of claims 1 to 14, further comprising one or more pharmaceutically acceptable vehicles, diluents and/or excipients and/or one or more adjuvants.

**16.** The artificial nucleic acid molecule as defined by any one of claims 1-11, the vector as defined by claim 12 or 13, the cell as defined by claim 14, or the pharmaceutical composition according to any one of claims 1 to 15 for use as a medicament.

**17.** The artificial nucleic acid molecule as defined by any one of claims 1-11, the vector as defined by claim 12 or 13, the cell as defined by claim 14, or the pharmaceutical composition according to any one of claims 1 to 15 for use as a vaccine or for use in gene therapy.

**18.** An *in vitro* method for stabilizing and/or prolonging protein production from an artificial nucleic acid molecule, preferably from an mRNA molecule or a vector, the *in vitro* method comprising the step of associating the nucleic acid molecule, preferably the mRNA molecule or the vector, with an 3'-UTR element, wherein the 3'-UTR element comprises or consists of a nucleic acid sequence according to SEQ ID NO: 1 or 2, or a functional fragment or a functional variant thereof, wherein the functional fragment or the functional variant has an identity of at least 60% to the nucleic acid sequence according to SEQ ID NO: 1 or 2, wherein the artificial nucleic acid comprises an open reading frame and wherein the open reading frame and the 3'-UTR element are heterologous to each other.

**19.** Use of an 3'-UTR element for stabilizing and/or prolonging protein production from a nucleic acid molecule, preferably from an mRNA molecule or a vector, wherein the 3'-UTR element comprises or consists of a nucleic acid sequence according to SEQ ID NO: 1 or 2, or a functional fragment or a functional variant thereof, wherein the functional fragment or the functional variant has an identity of at least 60% to the nucleic acid sequence according to SEQ ID NO: 1 or 2, wherein the nucleic acid comprises an open reading frame and wherein the open reading frame and the 3'-UTR element are heterologous to each other.

**20.** A kit or kit of parts comprising a pharmaceutical composition according to any one of claims 1 to 15.

**21.** The kit according to claim 20 further comprising instructions for use, cells for transfection, an adjuvant, means for administration of the pharmaceutical composition, a pharmaceutically acceptable carrier and/or a pharmaceutically acceptable solution for dissolution or dilution of the pharmaceutical composition.

**Patentansprüche**

**1.** Pharmazeutische Zusammensetzung umfassend ein artifizielles Nukleinsäuremolekül, welches umfasst:

a. mindestens einen offenen Leserahmen (ORF); und

b. mindestens ein 3'-UTR-Element (3'-untranslatierte Region), welches eine Nukleinsäuresequenz gemäß SEQ ID NO: 1 oder 2 oder ein funktionelles Fragment oder eine funktionelle Variante davon umfasst, wobei das funktionelle Fragment oder die funktionelle Variante eine Identität von mindestens 60% mit der Nukleinsäuresequenz gemäß SEQ ID NO: 1 oder 2 aufweisen,

wobei der offene Leserahmen (ORF) und das 3'-UTR-Element zueinander heterolog sind.

**2.** Pharmazeutische Zusammensetzung gemäß Anspruch 1, wobei der offene Leserahmen (ORF) kein Reportergen kodiert oder nicht von einem Reportergen abgeleitet ist, wobei das Reportergen vorzugsweise nicht aus der Gruppe bestehend aus lichtemittierenden Proteinen, vorzugsweise nicht Luciferase, fluoreszierenden Proteinen, vorzugsweise nicht rot-, blau- oder grün-fluoreszierenden Proteinen; enzymatischen Reportern; dem lacZ-Gen von *E. coli* (beta-Galaktosidase); Alkalischer Phosphatase; sekretierter embryonaler alkalischer Phosphatase (SEAP); Chloramphenicol-Acetyl-Transferase (CAT); Hormonen und Zytokinen ausgewählt ist.

**3.** Pharmazeutische Zusammensetzung gemäß Anspruch 1 oder 2, wobei der offene Leserahmen (ORF) kein FIG4-Gen, bevorzugt kein eukaryotisches FIG4-Gen, bevorzugter kein Säugetier-FIG4-Gen, am meisten bevorzugt kein humanes FIG4-Gen, kodiert oder nicht von einem solchen FIG4-Gen abgeleitet ist.

**4.** Pharmazeutische Zusammensetzung gemäß irgendeinem der Ansprüche 1 bis 3, wobei das mindestens eine 3'-UTR-Element die Proteinproduktion von dem artifiziellen Nukleinsäuremolekül stabilisiert/verlängert.

**5.** Pharmazeutische Zusammensetzung gemäß irgendeinem der Ansprüche 1 bis 4, wobei das mindestens eine 3'-UTR-Element eine Nukleinsäuresequenz umfasst oder aus dieser besteht, die eine Identität von mindestens etwa 70%, bevorzugt von mindestens etwa 80%, bevorzugter von mindestens etwa 90%, noch bevorzugter von mindestens etwa 95%, noch bevorzugter von mindestens etwa 99% mit der Nukleinsäuresequenz gemäß SEQ ID NO: 1 oder 2 aufweist, oder wobei das mindestens eine 3'-UTR-Element ein Fragment einer Nukleinsäuresequenz umfasst oder daraus besteht, welches eine Identität von mindestens etwa 70%, bevorzugt von mindestens etwa 80%, bevorzugter von mindestens etwa 90%, noch bevorzugter von mindestens etwa 95%, noch bevorzugter von mindestens etwa 99% mit der Nukleinsäuresequenz gemäß SEQ ID NO: 1 oder 2 aufweist.

**6.** Pharmazeutische Zusammensetzung gemäß irgendeinem der Ansprüche 1 bis 5, wobei das artifizielle Nukleinsäuremolekül ferner umfasst:

c. eine Poly(A)-Sequenz und/oder ein Polyadenylierungssignal, wobei sich die Poly(A)-Sequenz oder das Polyadenylierungssignal vorzugsweise 3' des 3'-UTR-Elements befinden.

**7.** Pharmazeutische Zusammensetzung gemäß irgendeinem der Ansprüche 1 bis 6, wobei das artifizielle Nukleinsäuremolekül ferner eine 5'-Cap-Struktur, eine Poly(C)-Sequenz, eine Histon-Stamm-Schleife (Stem-Loop) und/oder ein IRES-Motiv umfasst.

**8.** Pharmazeutische Zusammensetzung gemäß irgendeinem der Ansprüche 1 bis 7, wobei die Nukleinsäure ein zusätzliches 5'-UTR-Element, vorzugsweise eine 5'-UTR, einen Promotor oder eine 5'-UTR und eine einen Promotor enthaltende Sequenz umfasst.

**9.** Pharmazeutische Zusammensetzung gemäß Anspruch 8, wobei die 5'-UTR eine 5'-TOP-UTR ist.

**10.** Pharmazeutische Zusammensetzung gemäß irgendeinem der Ansprüche 1 bis 9, wobei das artifizielle Nukleinsäuremolekül, vorzugsweise der offene Leserahmen, wenigstens teilweise G/C-modifiziert ist, wobei bevorzugt der G/C-Gehalt des offenen Leserahmens im Vergleich zu dem offenen Leserahmen des Wildtyps erhöht ist, und/oder wobei der offene Leserahmen eine Codon-optimierte Region umfasst, wobei vorzugsweise der offene Leserahmen Codon-optimiert ist.

**11.** Pharmazeutische Zusammensetzung gemäß irgendeinem der Ansprüche 1 bis 10, wobei das artifizielle Nukleinsäuremolekül eine RNA, vorzugsweise ein mRNA-Molekül, ist.

**12.** Pharmazeutische Zusammensetzung umfassend einen Vektor, welcher das artifizielle Nukleinsäuremolekül, wie in irgendeinem der Ansprüche 1 bis 11 definiert, umfasst.

**13.** Pharmazeutische Zusammensetzung gemäß Anspruch 12, wobei der Vektor ein Plasmid-Vektor oder ein viraler Vektor, vorzugsweise ein Plasmid-Vektor, ist.

**14.** Pharmazeutische Zusammensetzung gemäß irgendeinem der Ansprüche 1 bis 13, umfassend eine Zelle, welche das artifizielle Nukleinsäuremolekül, wie in irgendeinem der Ansprüche 1 bis 11 definiert, oder den Vektor, wie in Anspruch 12 oder 13 definiert, umfasst.

**15.** Pharmazeutische Zusammensetzung gemäß irgendeinem der Ansprüche 1 bis 14, welche außerdem einen oder mehrere pharmazeutisch akzeptable Trägerstoffe, Verdünnungsmittel und/oder Hilfsstoffe und/oder ein Adjuvans oder mehrere Adjuvanzien umfasst.

**16.** Artifizielles Nukleinsäuremolekül, wie in irgendeinem der Ansprüche 1 bis 11 definiert, Vektor, wie in Anspruch 12 oder 13 definiert, Zelle, wie in Anspruch 14 definiert, oder pharmazeutische Zusammensetzung gemäß irgendeinem der Ansprüche 1 bis 15 zur Verwendung als Medikament.

**17.** Artifizielles Nukleinsäuremolekül, wie in irgendeinem der Ansprüche 1 bis 11 definiert, Vektor, wie in Anspruch 12 oder 13 definiert, Zelle, wie in Anspruch 14 definiert, oder pharmazeutische Zusammensetzung gemäß irgendeinem der Ansprüche 1 bis 15 zur Verwendung als Vakzine oder zur Verwendung in der Gentherapie.

**18.** *In-vitro*-Verfahren zur Stabilisierung und/oder Verlängerung der Proteinproduktion von einem artifiziellen Nukleinsäuremolekül, vorzugsweise von einem mRNA-Molekül oder einem Vektor, wobei das *In-vitro*-Verfahren den Schritt des Verbindens des Nukleinsäuremoleküls, vorzugsweise des mRNA-Moleküls oder des Vektors, mit einem 3'-UTR-Element umfasst, wobei das 3'-UTR-Element eine Nukleinsäuresequenz gemäß SEQ ID NO: 1 oder 2 oder ein funktionelles Fragment oder eine funktionelle Variante davon umfasst oder daraus besteht, wobei das funktionelle Fragment oder die funktionelle Variante eine Identität von mindestens 60% mit der Nukleinsäuresequenz gemäß SEQ ID NO: 1 oder 2 aufweisen, wobei das artifizielle Nukleinsäuremolekül einen offenen Leserahmen umfasst, und wobei der offene Leserahmen und das 3'-UTR-Element zueinander heterolog sind.

**19.** Verwendung eines 3'-UTR-Elements zur Stabilisierung und/oder Verlängerung der Proteinproduktion von einem Nukleinsäuremolekül, vorzugsweise von einem mRNA-Molekül oder einem Vektor, wobei das 3'-UTR-Element eine Nukleinsäuresequenz gemäß SEQ ID NO: 1 oder 2 oder ein funktionelles Fragment oder eine funktionelle Variante davon umfasst oder daraus besteht, wobei das funktionelle Fragment oder die funktionelle Variante eine Identität von mindestens 60% mit der Nukleinsäuresequenz gemäß SEQ ID NO: 1 oder 2 aufweisen, wobei das Nukleinsäuremolekül einen offenen Leserahmen umfasst, und wobei der offene Leserahmen und das 3'-UTR-Element zueinander heterolog sind.

**20.** Kit oder Kit aus Einzelbestandteilen (Kit of Parts), umfassend eine pharmazeutische Zusammensetzung gemäß irgendeinem der Ansprüche 1 bis 15.

**21.** Kit gemäß Anspruch 20, welcher außerdem Gebrauchsanweisungen, Zellen für die Transfektion, ein Adjuvans, Mittel zur Verabreichung der pharmazeutischen Zusammensetzung, einen pharmazeutisch akzeptablen Trägerstoff und/oder eine pharmazeutische akzeptable Lösung zur Auflösung oder Verdünnung der pharmazeutischen Zusammensetzung umfasst.

## Revendications

**1.** Composition pharmaceutique comprenant une molécule artificielle d'acide nucléique comprenant

a. au moins un cadre de lecture ouvert (ORF) ; et
b. au moins un élément de région non traduite 3' (élément 3'-UTR) comprenant une séquence d'acides nucléiques selon SEQ ID n° : 1 ou 2, ou son fragment fonctionnel ou son variant fonctionnel, le fragment fonctionnel ou le variant fonctionnel ayant une identité d'au moins 60 % vis-à-vis de la séquence d'acides nucléiques selon SEQ ID no : 1 ou 2,

le cadre de lecture ouvert (ORF) et l'élément 3'-UTR étant hétérologues l'un vis-à-vis de l'autre.

**2.** Composition pharmaceutique selon la revendication 1, le cadre de lecture ouvert (ORF) ne codant pas pour un

gène rapporteur ou ne dérivant pas d'un gène rapporteur, le gène rapporteur n'étant préférablement pas sélectionné dans le groupe constitué des protéines luminescentes, préférablement pas la luciférase, des protéines fluorescentes, préférablement pas les protéines fluorescentes rouge, bleue ou verte ; des rapporteurs enzymatiques ; du gène lacZ provenant d'*E. coli* (bêta-galactosidase) ; de la phosphatase alcaline ; de la phosphatase alcaline embryonnaire sécrétée (SEAP) ; de la chloramphénicol acétyl transférase (CAT) ; des hormones et des cytokines.

**3.** Composition pharmaceutique selon la revendication 1 ou 2, le cadre de lecture ouvert (ORF) ne codant pas pour un gène FIG4 ni n'étant dérivé d'un gène FIG4, préférablement pas d'un gène FIG4 eucaryote, plus préférablement pas d'un gène FIG4 de mammifère, non de manière préférable entre toutes d'un gène FIG4 humain.

**4.** Composition pharmaceutique selon l'une quelconque des revendications 1 à 3, ledit élément 3'-UTR stabilisant/prolongeant la production de protéine à partir de ladite molécule artificielle d'acide nucléique.

**5.** Composition pharmaceutique selon l'une quelconque des revendications 1 à 4, ledit élément 3'-UTR comprenant ou étant constitué d'une séquence d'acides nucléiques, qui présente une identité d'au moins environ 70 %, préférablement d'au moins environ 80 %, plus préférablement d'au moins environ 90 %, même plus préférablement d'au moins environ 95 %, même plus préférablement d'au moins environ 99 % vis-à-vis de la séquence d'acides nucléiques selon SEQ ID n° : 1 ou SEQ ID n° : 2, ou ledit élément 3'-UTR comprend ou est constitué d'un fragment d'une séquence d'acides nucléiques, qui présente une identité d'au moins environ 70 %, préférablement d'au moins environ 80 %, plus préférablement d'au moins environ 90 %, même plus préférablement d'au moins environ 95 %, même plus préférablement d'au moins environ 99 % vis-à-vis de la séquence d'acides nucléiques selon SEQ ID n° : 1 ou SEQ ID n° : 2.

**6.** Composition pharmaceutique selon l'une quelconque des revendications 1 à 5, la molécule artificielle d'acide nucléique comprenant en outre :
c. une séquence poly(A) et/ou un signal de polyadénylation, la séquence poly(A) ou le signal de polyadénylation étant préférablement localisée en position 3' de l'élément 3'-UTR.

**7.** Composition pharmaceutique selon l'une quelconque des revendications 1 à 6, la molécule artificielle d'acide nucléique comprenant en outre une structure de coiffage en 5', une séquence poly(C), une tige-boucle histone, et/ou un motif IRES.

**8.** Composition pharmaceutique selon l'une quelconque des revendications 1 à 7, l'acide nucléique comprenant un élément 5' additionnel, préférablement une 5'-UTR, un promoteur, ou une 5'-UTR et une séquence contenant un promoteur.

**9.** Composition pharmaceutique selon la revendication 8, la 5'-UTR étant une 5'-TOP UTR.

**10.** Composition pharmaceutique selon l'une quelconque des revendications 1 à 9, la molécule artificielle d'acide nucléique, préférablement le cadre de lecture ouvert, étant au moins partiellement G/C modifié, préférablement la teneur en G/C du cadre de lecture ouvert étant accrue comparée au cadre de lecture ouvert de type sauvage, et/ou le cadre de lecture ouvert comprenant une région à codon optimisé, préférablement, le cadre de lecture ouvert étant à codon optimisé.

**11.** Composition pharmaceutique selon l'une quelconque des revendications 1 à 10, la molécule artificielle d'acide nucléique étant un ARN, préférablement une molécule d'ARNm.

**12.** Composition pharmaceutique comprenant un vecteur comprenant la molécule artificielle d'acide nucléique telle que définie selon l'une quelconque des revendications 1 à 11.

**13.** Composition pharmaceutique selon la revendication 12, le vecteur étant un vecteur plasmide ou un vecteur viral, préférablement un vecteur plasmide.

**14.** Composition pharmaceutique selon l'une quelconque des revendications 1 à 13 comprenant une cellule comprenant la molécule artificielle d'acide nucléique telle que définie selon l'une quelconque des revendications 1 à 11 ou comprenant le vecteur tel que défini selon la revendication 12 ou 13.

**15.** Composition pharmaceutique selon l'une quelconque des revendications 1 à 14, comprenant en outre un ou plusieurs

véhicules, diluants et/ou excipients et/ou adjuvants pharmaceutiquement acceptables.

**16.** Molécule artificielle d'acide nucléique telle que définie selon l'une quelconque des revendications 1 à 11, vecteur tel que défini selon la revendication 12 ou 13, cellule telle que définie selon la revendication 14, ou composition pharmaceutique selon l'une quelconque des revendications 1 à 15 destiné-e à une utilisation comme médicament.

**17.** Molécule artificielle d'acide nucléique telle que définie selon l'une quelconque des revendications 1 à 11, vecteur tel que défini selon la revendication 12 ou 13, cellule telle que définie selon la revendication 14, ou composition pharmaceutique selon l'une quelconque des revendications 1 à 15 destiné-e à une utilisation comme vaccin ou une utilisation en thérapie génique.

**18.** Procédé *in vitro* de stabilisation et/ou de prolongation de la production de protéine à partir d'une molécule artificielle d'acide nucléique, préférablement à partir d'une molécule d'ARNm ou d'un vecteur, le procédé *in vitro* comprenant l'étape d'association de la molécule d'acide nucléique, préférablement la molécule d'ARNm ou le vecteur, avec un élément 3'-UTR, l'élément 3'-UTR comprenant ou étant constitué d'une séquence d'acides nucléiques selon SEQ ID n° : 1 ou 2, ou son fragment fonctionnel ou son variant fonctionnel, le fragment fonctionnel ou le variant fonctionnel ayant une identité d'au moins 60 % vis-à-vis de la séquence d'acides nucléiques selon SEQ ID n° : 1 ou 2, l'acide nucléique artificiel comprenant un cadre de lecture ouvert et le cadre de lecture ouvert et l'élément 3'-UTR étant hétérologues l'un vis-à-vis de l'autre.

**19.** Utilisation d'un élément 3'-UTR pour la stabilisation et/ou la prolongation de la production de protéine à partir d'une molécule d'acide nucléique, préférablement d'une molécule d'ARNm ou d'un vecteur, l'élément 3'-UTR comprenant ou étant constitué d'une séquence d'acides nucléiques selon SEQ ID n° : 1 ou 2, ou son fragment fonctionnel ou son variant fonctionnel, le fragment fonctionnel ou le variant fonctionnel ayant une identité d'au moins 60 % vis-à-vis de la séquence d'acides nucléiques selon SEQ ID n° : 1 ou 2, l'acide nucléique comprenant un cadre de lecture ouvert et le cadre de lecture ouvert et l'élément 3'-UTR étant hétérologues l'un vis-à-vis de l'autre.

**20.** Kit ou kit de parties comprenant une composition pharmaceutique selon l'une quelconque des revendications 1 à 15.

**21.** Kit selon la revendication 20 comprenant en outre des instructions d'utilisation, des cellules destinées à la transfection, un adjuvant, un moyen d'administration de la composition pharmaceutique, un support pharmaceutiquement acceptable et/ou une solution pharmaceutiquement acceptable pour la dissolution ou la dilution de la composition pharmaceutique.

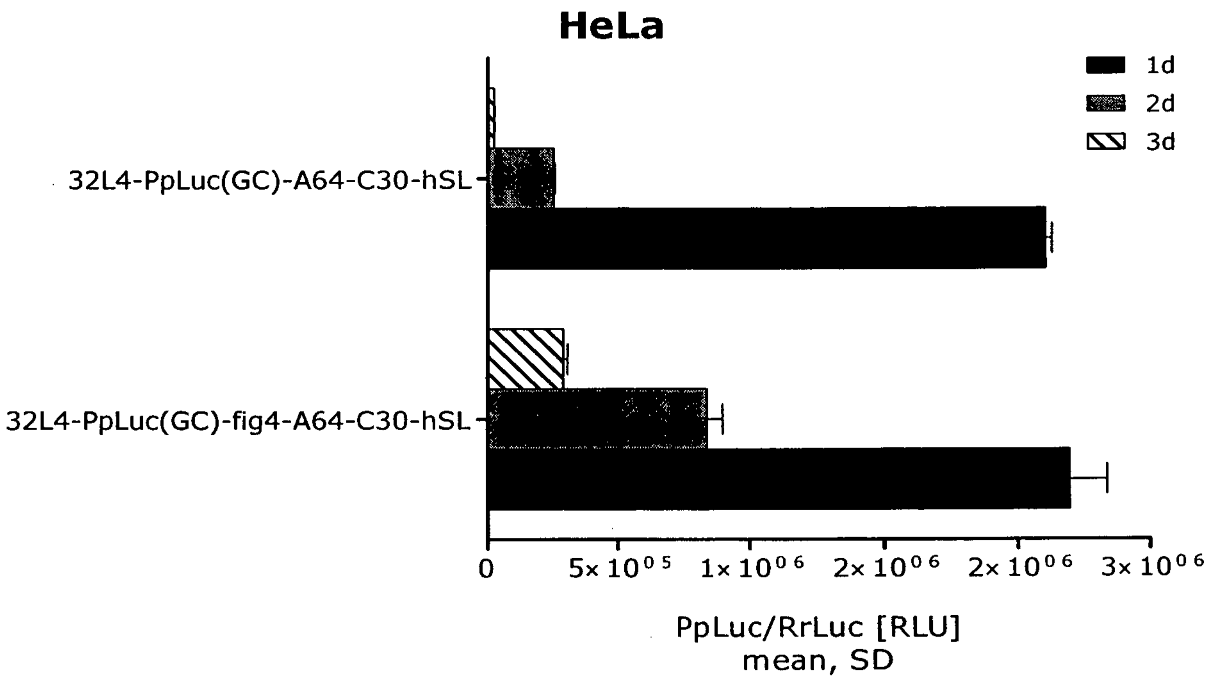
HeLa
1d
2d
3d
32L4-PpLuc(GC)-A64-C30-hSL
32L4-PpLuc(GC)-fig4-A64-C30-hSL
0
5× 10⁰⁵
1× 10⁰⁶
2× 10⁰⁶
2× 10⁰⁶
3× 10⁰⁶
PpLuc/RrLuc [RLU]
mean, SD

Fig. 1

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUAGAUCUAAAAAAAAAAAAAAAAAAAAA
AAAAAAAAAAAAAAAAAAAAAAAAAAAAAAAAAAAAAAAAAAAUGCAUCCCCCCCCCC
CCCCCCCCCCCCCCCCCCCCAAAGGCUCUUUUCAGAGCCACCAGAAUU

Fig. 2

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUAAAGAGCGCAGGUCCACCUGGUGGAC
ACGUCUGAUUAGCUUAGAACCUGUCUUGUCUCAUCUUCAAAAGGUAACUUAUUAAAAGUC
CUUUGCGUCUGAAGCCUUUCUCCUUUUCUGUCACUUGCAAAUUCCAAAUUAUAGCUAAUA
AAGAUGACUAGAUAAUUUGCAGAUCUAAAAAAAAAAAAAAAAAAAAAAAAAAAAAAAAAA
AAAAAAAAAAAAAAAAAAAAAAAAAAAAAAUGCAUCCCCCCCCCCCCCCCCCCCCCCCCC
CCCCCCAAAGGCUCUUUUCAGAGCCACCAGAAUU

Fig. 3

PpLuc(GC) – A64 - C30 - hSL. (SEQ ID NO: 9)

GGGAGAAAGCTTGAGGATGGAGGACGCCAAGAACATCAAGAAGGGCCCGGCGCCCTTCTA
CCCGCTGGAGGACGGGACCGCCGGCGAGCAGCTCCACAAGGCCATGAAGCGGTACGCCCT
GGTGCCGGGCACGATCGCCTTCACCGACGCCCACATCGAGGTCGACATCACCTACGCGGA
GTACTTCGAGATGAGCGTGCGCCTGGCCGAGGCCATGAAGCGGTACGGCCTGAACACCAA
CCACCGGATCGTGGTGTGCTCGGAGAACAGCCTGCAGTTCTTCATGCCGGTGCTGGGCGC
CCTCTTCATCGGCGTGGCCGTCGCCCCGGCGAACGACATCTACAACGAGCGGGAGCTGCT
GAACAGCATGGGGATCAGCCAGCCGACCGTGGTGTTCGTGAGCAAGAAGGGCCTGCAGAA
GATCCTGAACGTGCAGAAGAAGCTGCCCATCATCCAGAAGATCATCATCATGGACAGCAA
GACCGACTACCAGGGCTTCCAGTCGATGTACACGTTCGTGACCAGCCACCTCCCGCCGGG
CTTCAACGAGTACGACTTCGTCCCGGAGAGCTTCGACCGGGACAAGACCATCGCCCTGAT
CATGAACAGCAGCGGCAGCACCGGCCTGCCGAAGGGGGTGGCCCTGCCGCACCGGACCGC
CTGCGTGCGCTTCTCGCACGCCCGGGACCCCATCTTCGGCAACCAGATCATCCCGGACAC
CGCCATCCTGAGCGTGGTGCCGTTCCACCACGGCTTCGGCATGTTCACGACCCTGGGCTA
CCTCATCTGCGGCTTCCGGGTGGTCCTGATGTACCGGTTCGAGGAGGAGCTGTTCCTGCG
GAGCCTGCAGGACTACAAGATCCAGAGCGCGCTGCTCGTGCCGACCCTGTTCAGCTTCTT
CGCCAAGAGCACCCTGATCGACAAGTACGACCTGTCGAACCTGCACGAGATCGCCAGCGG
GGGCGCCCCGCTGAGCAAGGAGGTGGGCGAGGCCGTGGCCAAGCGGTTCCACCTCCCGGG
CATCCGCCAGGGCTACGGCCTGACCGAGACCACGAGCGCGATCCTGATCACCCCCGAGGG
GGACGACAAGCCGGGCGCCGTGGGCAAGGTGGTCCCGTTCTTCGAGGCCAAGGTGGTGGA
CCTGGACACCGGCAAGACCCTGGGCGTGAACCAGCGGGGCGAGCTGTGCGTGCGGGGGCC
GATGATCATGAGCGGCTACGTGAACAACCCGGAGGCCACCAACGCCCTCATCGACAAGGA
CGGCTGGCTGCACAGCGGCGACATCGCCTACTGGGACGAGGACGAGCACTTCTTCATCGT
CGACCGGCTGAAGTCGCTGATCAAGTACAAGGGCTACCAGGTGGCGCCGGCCGAGCTGGA
GAGCATCCTGCTCCAGCACCCCAACATCTTCGACGCCGGCGTGGCCGGGCTGCCGGACGA
CGACGCCGGCGAGCTGCCGGCCGCGGTGGTGGTGCTGGAGCACGGCAAGACCATGACGGA
GAAGGAGATCGTCGACTACGTGGCCAGCCAGGTGACCACCGCCAAGAAGCTGCGGGGCGG
CGTGGTGTTCGTGGACGAGGTCCCGAAGGGCCTGACCGGGAAGCTCGACGCCCGGAAGAT
CCGCGAGATCCTGATCAAGGCCAAGAAGGGCGGCAAGATCGCCGTGTAAGACTAGTAGAT
CTAAAAAAAAAAAAAAAAAAAAAAAAAAAAAAAAAAAAAAAAAAAAAAAAAAAAAAAAAA
AAAAAATGCATCCCCCCCCCCCCCCCCCCCCCCCCCCCCCCAAAGGCTCTTTTCAGAGC
CACCAGAATT

Fig. 4

PpLuc(GC) – fig4-A64-C30-hSL. (SEQ ID NO: 10).

GGGAGAAAGCUUGAGG*AUGGAGGACGCCAAGAACAUCAAGAAGGGCCCGGCGCCCUUCUA*
CCCGCUGGAGGACGGACCGCCGGCGAGCAGCUCCACAAGGCCAUGAAGCGGUACGCCCU
GGUGCCGGGCACGAUCGCCUUCACCGACGCCCACAUCGAGGUCGACAUCACCUACGCGGA
GUACUUCGAGAUGAGCGUGCGCCUGGCCGAGGCCAUGAAGCGGUACGGCCUGAACACCAA
CCACCGGAUCGUGGUGUGCUCGGAGAACAGCCUGCAGUUCUUCAUGCCGGUGCUGGGCGC
CCUCUUCAUCGGCGUGGCCGUCGCCCCGGCGAACGACAUCUACAACGAGCGGGAGCUGCU
GAACAGCAUGGGGAUCAGCCAGCCGACCGUGGUGUUCGUGAGCAAGAAGGGCCUGCAGAA
GAUCCUGAACGUGCAGAAGAAGCUGCCCAUCAUCCAGAAGAUCAUCAUCAUGGACAGCAA
GACCGACUACCAGGGCUUCCAGUCGAUGUACACGUUCGUGACCAGCCACCUCCCGCCGGG
CUUCAACGAGUACGACUUCGUCCCGGAGAGCUUCGACCGGGACAAGACCAUCGCCCUGAU
CAUGAACAGCAGCGGCAGCACCGGCCUGCCGAAGGGGGUGGCCCUGCCGCACCGGACCGC
CUGCGUGCGCUUCUCGCACGCCCGGGACCCCAUCUUCGGCAACCAGAUCAUCCCGGACAC
CGCCAUCCUGAGCGUGGUGCCGUUCCACCACGGCUUCGGCAUGUUCACGACCCUGGGCUA
CCUCAUCUGCGGCUUCCGGGUGGUCCUGAUGUACCGGUUCGAGGAGGAGCUGUUCCUGCG
GAGCCUGCAGGACUACAAGAUCCAGAGCGCGCUGCUCGUGCCGACCCUGUUCAGCUUCUU
CGCCAAGAGCACCCUGAUCGACAAGUACGACCUGUCGAACCUGCACGAGAUCGCCAGCGG
GGGCGCCCCGCUGAGCAAGGAGGUGGGCGAGGCCGUGGCCAAGCGGUUCCACCUCCCGGG
CAUCCGCCAGGGCUACGGCCUGACCGAGACCACGAGCGCGAUCCUGAUCACCCCCGAGGG
*GGACGACAAGCCGGGCGCCGUGGGCAAGGUGGUCCCGUUCUUCGAGGCCAAGGUGGUGGA*
*CCUGGACACCGGCAAGACCCUGGGCGUGAACCAGCGGGGCGAGCUGUGCGUGCGGGGCC*
*GAUGAUCAUGAGCGGCUACGUGAACAACCCGGAGGCCACCAACGCCCUCAUCGACAAGGA*
*CGGCUGGCUGCACAGCGGCGACAUCGCCUACUGGGACGAGGACGAGCACUUCUUCAUCGU*
*CGACCGGCUGAAGUCGCUGAUCAAGUACAAGGGCUACCAGGUGGCGCCGGCCGAGCUGGA*
*GAGCAUCCUGCUCCAGCACCCCAACAUCUUCGACGCCGGCGUGGCCGGGCUGCCGGACGA*
*CGACGCCGGCGAGCUGCCGGCCGCGGUGGUGGUGCUGGAGCACGGCAAGACCAUGACGGA*
*GAAGGAGAUCGUCGACUACGUGGCCAGCCAGGUGACCACCGCCAAGAAGCUGCGGGGCGG*
*CGUGGUGUUCGUGGACGAGGUCCCGAAGGGCCUGACCGGGAAGCUCGACGCCCGGAAGAU*
*CCGCGAGAUCCUGAUCAAGGCCAAGAAGGGCGGCAAGAUCGCCGUGUAA*GACUAGUAAAG
AGCGCAGGUCCACCUGGUGGACACGUCUGAUUAGCUUAGAACCUGUCUUGUCUCAUCUUC
AAAAGGUAACUUAUUAAAAGUCCUUUGCGUCUGAAGCCUUUCUCCUUUUCUGUCACUUGC
AAAUUCCAAAUUAUAGCUAAUAAAGAUGACUAGAUAAUUUGCAGAUCUAAAAAAAAAAAA
AAAAAAAAAAAAAAAAAAAAAAAAAAAAAAAAAAAAAAAAAAAAAAAAAAAAUGCAUCCC
CCCCCCCCCCCCCCCCCCCCCCCCCCCAAAGGCUCUUUUCAGAGCCACCAGAAUU

Fig. 5

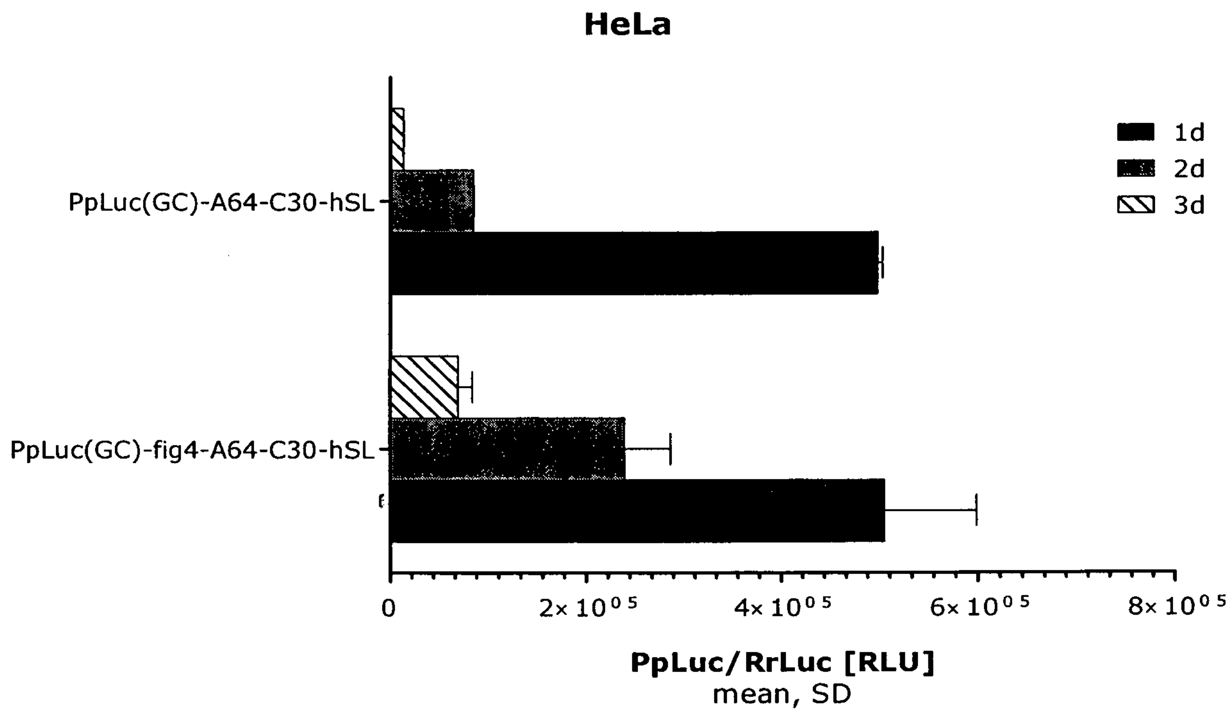
HeLa
PpLuc(GC)-A64-C30-hSL
PpLuc(GC)-fig4-A64-C30-hSL
1d
2d
3d
0
2×10^0 5
4×10^0 5
6×10^0 5
8×10^0 5
PpLuc/RrLuc [RLU]
mean, SD

Fig. 6

A.

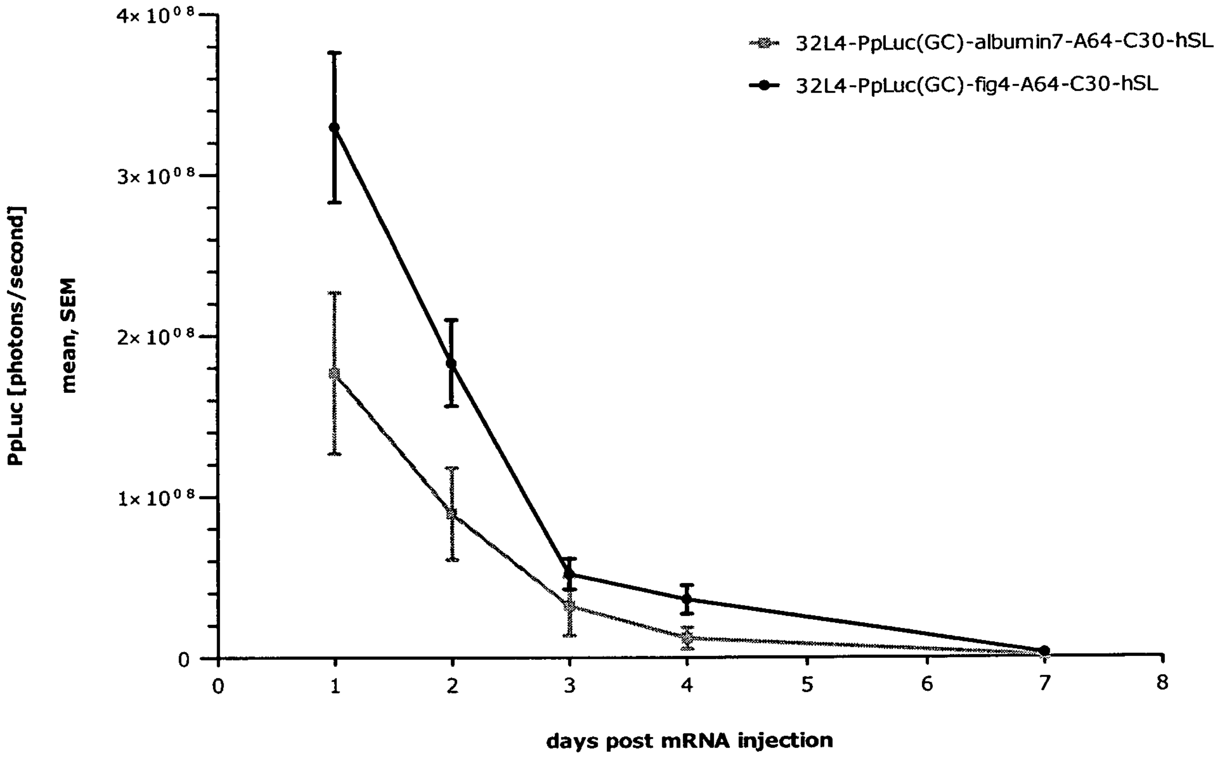
32L4-PpLuc(GC)-albumin7-A64-C30-hSL
32L4-PpLuc(GC)-fig4-A64-C30-hSL
PpLuc [photons/second]
mean, SEM
4× 10⁰⁸
3× 10⁰⁸
2× 10⁰⁸
1× 10⁰⁸
0
0
1
2
3
4
5
6
7
8
days post mRNA injection

B.

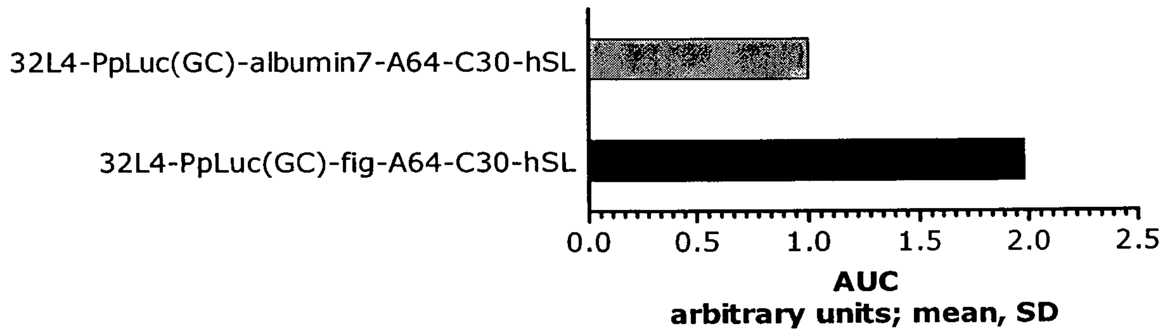
32L4-PpLuc(GC)-albumin7-A64-C30-hSL
32L4-PpLuc(GC)-fig-A64-C30-hSL
0.0
0.5
1.0
1.5
2.0
2.5
AUC
arbitrary units; mean, SD

Fig. 7

RPL32 – PpLuc(GC) – albumin7 – A64 – C30 – histoneSL (SEQ ID NO: 11)

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02098443 A
- WO 2008112127 A
- WO 2013120629 A
- WO 2008134539 A
- WO 2013143700 A
- WO 2012019780 A
- US 4373071 A
- US 4401796 A
- US 4415732 A
- US 4458066 A
- US 4500707 A
- US 4668777 A
- US 4973679 A
- US 5047524 A
- US 5132418 A
- US 5153319 A
- US 5262530 A
- US 5700642 A
- WO 2010037539 A
- WO 2013143698 A


### Non-patent literature cited in the description


- **FRIEDEL et al.** Conserved principles of mammalian transcriptional regulation revealed by RNA half-life. *Nucleic Acid Research,* 2009, vol. 37 (17), 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **RODGERS.** Regulated $\alpha$-globin mRNA decay is a cytoplasmic event proceeding through 3'-to-5' exosome-dependent decapping. *RNA,* 2002, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **MINAGAWA et al.** Identification and Characterization of a Sac Domain-containing Phosphoinositide 5-Phosphatase. *J. Biol. Chem.,* 2001, vol. 276, 22011-22015
- **TAKASUGA ; SASAKI.** Phosphatidylinositol-3,5-biphosphate: metabolism and physiological functions. *Journal of Biochemistry,* 2013, vol. 154 (3), 211-218
- **NAGASE et al.** Prediction of the coding sequences of unidentified human genes. VI. The coding sequences of 80 new genes (KIAA0201-KIAA0280) deduced by analysis of cDNA clones from cell line KG-1 and brain. *DNA Research,* 1996, vol. 3, 321-329
- **AKASHI.** *Curr. Opin. Genet. Dev.,* 2001, vol. 11 (6), 660-666
- **CAKMAKCI ; LERNER ; WAGNER ; ZHENG ; WILLIAM F MARZLUFF.** *Mol. Cell. Biol.,* 2008, vol. 28 (3), 1182-94